(19) Europäisches Patentamt
European Patent Office
Office européen des brevets

(11) **EP 1 411 818 B1**

(12) **EUROPEAN PATENT SPECIFICATION**

(45) Date of publication and mention
of the grant of the patent:
**17.02.2010 Bulletin 2010/07**

(51) Int Cl.:
***A61B 1/00*** (2006.01)

(21) Application number: **02755594.5**

(86) International application number:
**PCT/IL2002/000622**

(22) Date of filing: **26.07.2002**

(87) International publication number:
**WO 2003/009739 (06.02.2003 Gazette 2003/06)**

(54) **APPARATUS AND METHOD FOR CONTROLLING ILLUMINATION OR IMAGER GAIN IN AN IN-VIVO IMAGING DEVICE**

GERÄT UND VERFAHREN ZUR REGULIERUNG DER BELEUCHTUNG ODER BILDVERSTÄRKUNG IN EINER BILDGEBENDEN VORRICHTUNG IN VIVO

APPAREIL ET PROCEDE DE REGLAGE DE L'ECLAIREMENT ET DU GAIN D'IMAGEUR DANS UN DISPOSITIF D'IMAGERIE IN VIVO

(84) Designated Contracting States:
**AT BE BG CH CY CZ DE DK EE ES FI FR GB GR IE IT LI LU MC NL PT SE SK TR**

(30) Priority: **26.07.2001 US 307603 P**

(43) Date of publication of application:
**28.04.2004 Bulletin 2004/18**

(73) Proprietor: **Given Imaging Ltd.**
**20692 Yoqneam (IL)**

(72) Inventors:
• **AVNI, Dov**
**34525 Haifa (IL)**

• **GLUKHOVSKY, Arkady**
**Santa Clarita, CA 91355 (US)**

(74) Representative: **Siegert, Georg**
**Hoffmann - Eitle**
**Patent- und Rechtsanwälte**
**Arabellastrasse 4**
**81925 München (DE)**

(56) References cited:
**US-A- 4 278 077    US-A- 4 310 228**
**US-A- 5 604 531    US-A- 5 730 702**
**US-A- 5 833 603    US-B1- 6 240 312**

**Description**

**BACKGROUND OF THE INVENTION**

Devices and methods for performing in-vivo imaging of passages or cavities within a body are known in the art. Such devices may include, inter alia, various endoscopic imaging systems and devices for performing imaging in various internal body cavities.

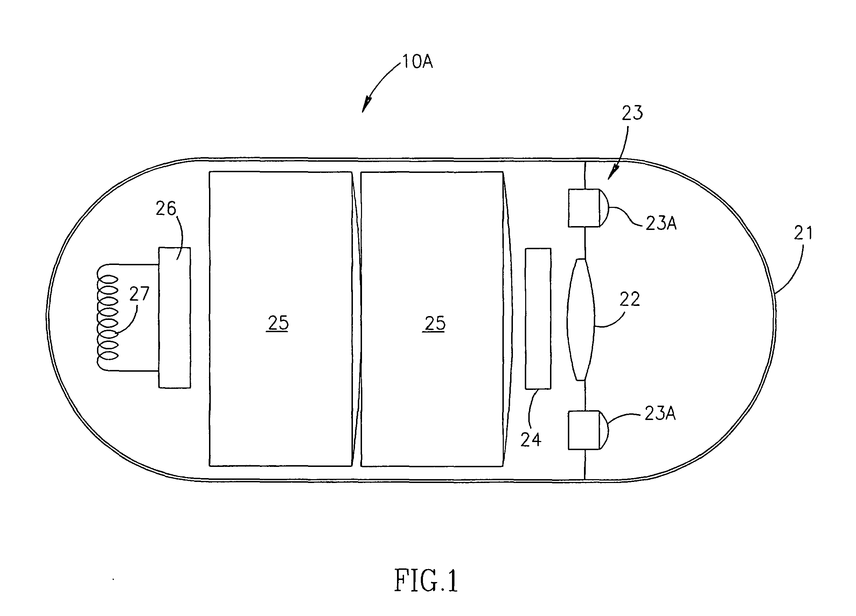

Reference is now made to Fig. 1 which is a schematic diagram illustrating an embodiment of an autonomous in-vivo imaging device. The device 10A typically includes an optical window 21 and an imaging system for obtaining images from inside a body cavity or lumen, such as the GI tract. The imaging system includes an illumination unit 23. The illumination unit 23 may include one or more discrete light sources 23A, or may include only one light source 23A. The one or more light sources 23A may be a white light emitting diode (LED), or any other suitable light source, known in the art. The device 10A includes a CMOS imaging sensor 24, which acquires the images and an optical system 22 which focuses the images onto the CMOS imaging sensor 24. The illumination unit 23 illuminates the inner portions of the body lumen through an optical window 21. Device 10A further includes a transmitter 26 and an antenna 27 for transmitting the video signal of the CMOS imaging sensor 24, and one or more power sources 25. The power source (s) 25 may be any suitable power sources such as but not limited to silver oxide batteries, lithium batteries, or other electrochemical cells having a high energy density, or the like. The power source(s) 25 may provide power to the electrical elements of the device 10A.

Typically, in the gastrointestinal application, as the device 10A is transported through the gastrointestinal (GI) tract, the imager, such as but not limited to the multi-pixel CMOS sensor 24 of the device 10A acquires images (frames) which are processed and transmitted to an external receiver/recorder (not shown) worn by the patient for recording and storage. The recorded data may then be downloaded from the receiver/recorder to a computer or workstation (not shown) for display and analysis, other systems and methods may also be suitable.

During the movement of the device 10A through the GI tract, the imager may acquire frames at a fixed or at a variable frame acquisition rate. For example, the imager (such as, but not limited to the CMOS sensor 24 of Fig. 1) may acquire images at a fixed rate of two frames per second (2 Hz). However, other different frame rates may also be used, depending, inter alia, on the type and characteristics of the specific imager or camera or sensor array implementation that is used, and on the available transmission bandwidth of the transmitter 26. The downloaded images may be displayed by the workstation by replaying them at a desired frame rate. This way, the expert or physician examining the data is provided with a movie-like video playback, which may enable the physician to review the passage of the device through the GI tract.

One of the limitations of electronic imaging sensors, is that they may have a limited dynamic range. The dynamic range of most existing electronic imaging sensors is significantly lower than the dynamic range of the human eye. Thus, when the imaged field of view includes both dark and bright parts of imaged objects, the limited dynamic range of the imaging sensor may result in underexposure of the dark parts of the field of view, or overexposure of the bright parts of the field of view, or both.

Various methods may be used for increasing the dynamic range of an imager. Such methods may include changing the amount of light reaching the imaging sensor, such as for example by changing the diameter of an iris or diaphragm included in the imaging device to increase or decrease the amount of light reaching the imaging sensor, methods for changing the exposure time, methods for changing the gain of the imager or methods for changing the intensity of the illumination. For example, in still cameras, the intensity of the flash unit may be changed during the exposure of the film.

When a series of consecutive frames is imaged such as in video cameras, the intensity of illumination of the imaged field of view within the currently imaged frame may be modified based on the results of measurement of light intensity performed in one or more previous frames. This method is based on the assumption that the illumination conditions do not change abruptly from one frame to the consecutive frame.

However, in an in vivo imaging device, for example, for imaging the GI tract, which operates at low frame rates and which is moved through a body lumen (e.g., propelled by the peristaltic movements of the intestinal walls), the illumination conditions may vary significantly from one frame to the next frame. Therefore, methods of controlling the illumination based on analysis of data or measurement results of previous frames may not be always feasible, particularly at low frame rates. An in vivo imaging device is know from US 6,240,312.

**SUMMARY OF THE INVENTION**

Embodiments of the present invention include a device and method for operating an in vivo imaging device wherein the illumination produced by the device may be varied in intensity and/or duration according to, for example, the amount of illumination produced by the device, which is reflected back to the device. In such a manner, the illumination can be controlled and made more efficient. The invention is defined in the independent claims.

## BRIEF DESCRIPTION OF THE DRAWINGS

The invention is herein described, by way of example only, with reference to the accompanying drawings, in which like components are designated by like reference numerals, wherein:

Fig. 1 is a schematic diagram illustrating an embodiment of a prior art autonomous in-vivo imaging device;

Fig. 2 is a schematic block diagram illustrating part of an in-vivo imaging device having an automatic illumination control system, in accordance with an embodiment of the present invention;

Fig. 3 is a schematic cross-sectional view of part of an in-vivo imaging device having an automatic illumination control system and four light sources, in accordance with an embodiment of the present invention;

Fig. 4 is a schematic front view of the device illustrated in Fig. 3;

Fig. 5 is a schematic diagram illustrating a method of timing of the illumination and image acquisition in an in vivo imaging device having a fixed illumination duration, according to an embodiment of the invention;

Fig. 6 is a schematic diagram illustrating one possible configuration for an illumination control unit coupled to a light sensing photodiode and to a light emitting diode, in accordance with an embodiment of the present invention;

Fig. 7 is a schematic diagram illustrating the illumination control unit of Fig. 6 in detail, in accordance with an embodiment of the present invention;

Fig. 8 is a schematic diagram useful for understanding a method of timing of the illumination and image acquisition in an in vivo imaging device having a variable controlled illumination duration, according to an embodiment of the invention;

Fig. 9 is a schematic diagram useful for understanding a method of timing of the illumination and image acquisition in an in vivo imaging device having a variable frame rate and a variable controlled illumination duration according to an embodiment of the invention;

Fig 10A is a timing diagram schematically illustrating an imaging cycle of an in vivo imaging device using an automatic illumination control method, in accordance with another embodiment of the present invention;

Fig. 10B is a schematic exemplary graph representing the light intensity as a function of time, possible when using the method of automatic illumination control, according to an embodiment of the invention, illustrated in Fig. 10A;

Fig. 10C is another exemplary schematic graph representing another example of the light intensity as a function of time, possible when using the method of automatic illumination control, according to an embodiment of the invention, illustrated in Fig. 10A;

Fig. 11 is a schematic diagram illustrating an illumination control unit including a plurality of light sensing units for controlling a plurality of light sources, in accordance with an embodiment of the present invention;

Fig. 12 is a schematic diagram illustrating a front view of an autonomous imaging device having four light sensing units and four light sources, in accordance with an embodiment of the present invention;

Fig. 13 is a schematic top view illustrating the arrangement of pixels on the surface of a CMOS imager usable for illumination control, in accordance with an embodiment of the present invention;

Fig. 14 is a schematic top view of the pixels of a CMOS imager illustrating an exemplary distribution of control pixel groups suitable for being used in local illumination control in an imaging device, according to an embodiment of the invention;

Fig. 15A depicts a series of steps of a method according to an embodiment of the present invention; and

Fig. 15B depicts a series of steps of a method according to an alternate embodiment of the present invention.

## DETAILED DESCRIPTION OF THE INVENTION

Various aspects of the present invention are described herein. For purposes of explanation, specific configurations and details are set forth in order to provide a thorough understanding of the present invention. However, it will also be apparent to one skilled in the art that the present invention may be practiced without the specific details presented herein. Furthermore, well known features may be omitted or simplified in order not to obscure the present invention. The invention is defined in the independent claims.

Embodiments of the present invention are based, inter alia, on controlling the illumination provided by the in-vivo imaging device based on light measurement which is performed within the duration of a single frame acquisition time or a part thereof.

It is noted that while the embodiments of the invention shown hereinbelow are adapted for imaging of the gastrointestinal (GI) tract, the devices and methods disclosed herein may be adapted for imaging other body cavities or spaces.

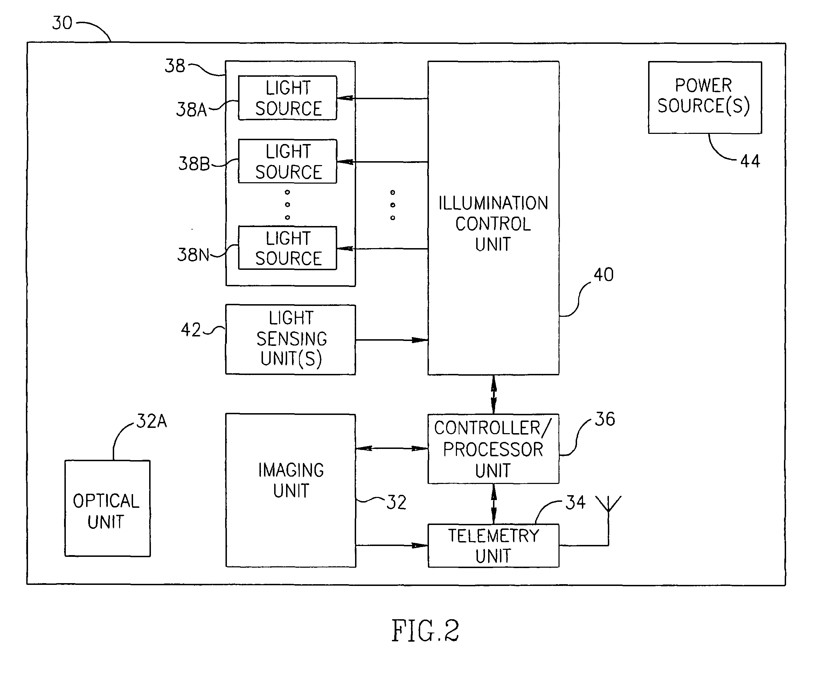

Reference is now made to Fig. 2 which is a schematic block diagram illustrating part of an in-vivo imaging device having an automatic illumination control system, in accordance with an embodiment of the present invention. The device 30 may be constructed as a swallowable video capsule as disclosed for the device 10A of Fig.1 or in U.S. patent 5,604,531 to Iddan et al., or in US 2001 035902 to Glukhovsky et al. However, the system and method of the present invention may be used in conjunction with other in-vivo imaging devices.

The device 30 may include an imaging unit 32 adapted for imaging the GI tract. The imaging unit 32 may include an imaging sensor (not shown in detail), such as but not limited to the CMOS imaging sensor 24 of Fig. 1. However, the imaging unit 32 may include any other suitable type of imaging sensor known in the art. The imaging unit 32 may also include an optical unit 32A including one or more optical elements (not shown), such as one or more lenses (not shown), one or more composite lens assemblies (not shown), one or more suitable optical filters (not shown), or any other suitable optical elements (not shown) adapted for focusing an image of the GI tract on the imaging sensor as is known in the art and disclosed hereinabove with respect to the optical unit 22 of Fig. 1.

The optical unit 32A may include one or more optical elements (not shown) which are integrated within the imaging unit 32, such as for example, a lens (not shown) which is attached to, or mounted on, or fabricated on or adjacent to the imager light sensitive pixels (not shown) as is known in the art.

The device 30 may also includes a telemetry unit 34 suitably connected to the imaging unit 32 for telemetrically transmitting the images acquired by the imaging unit 32 to an external receiving device (not shown), such as but not limited to the receiver/recorder device disclosed in U.S. patent 5,604,531 to Iddan et al., or in Co-pending US Patent Application, Serial No. 09/800,470 to Glukhovsky et al.

The device 30 also includes a controller/processor unit 36 suitably connected to the imaging unit 32 for controlling the operation of the imaging unit 32. The controller/processor unit 36 comprises any suitable type of controller, such as but not limited to, an analog controller, a digital controller such as, for example, a data processor, a microprocessor, a micro-controller, or a digital signal processor (DSP). The controller/processor unit 36 may also comprise hybrid analog/digital circuits as is known in the art. The controller/ processor unit 36 may be suitably connected to the telemetry unit 34 for controlling the transmission of image frames by the telemetry unit 34.

The controller/processor unit 36 may be (optionally) suitably connected to the imaging unit 32 for sending control signals thereto. The controller/processor unit 36 may thus (optionally) control the transmission of image data from the imaging unit 32 to the telemetry unit 34.

The device 30 may include an illuminating unit 38 for illuminating the GI tract. The illuminating unit 38 may include one or more discrete light sources 38A, 38B, to 38N or may include only one light source; such light source(s) may be, for example, but are not limited to, the light sources 23A of Fig. 1. The light source(s) 38A, 38B, to 38N of the illuminating unit 38 may be white light emitting diodes, such as the light sources disclosed in co-pending US Patent Application, Serial No. 09/800,470 to Glukhovsky et al. However, the light source(s) 38A, 38B. 38N of the illuminating unit 38 may also be any other suitable light source, known in the art, such as but not limited to incandescent lamp(s), flash lamp(s) or gas discharge lamp(s), or any other suitable light source(s).

It is noted that, in accordance with another embodiment of the present invention, the in vivo imaging device may include a single light source (not shown).

The device 30 also includes an illumination control unit 40 suitably connected to the light sources 38A, 38B, to 38N of the illuminating unit 38 for controlling the energizing of the light sources 38A, 38B, to 38N of the illuminating unit 38. The illumination control unit 40 may be used for switching one or more of the light sources 38A, 38B, to 38N on or off, or for controlling the intensity of the light produced by one or more of the light sources 38A, 38B, to 38N, as is disclosed in detail hereinafter.

The controller/processor unit 36 may be suitably connected to the illumination control unit 40 for (optionally) sending control signals thereto. Such control signals may be used for synchronizing or timing the energizing of the light sources 38A, 38B, to 38N within the illuminating unit 38, relative to the imaging cycle or period of the imaging unit 32. The illumination control unit 40 may be (optionally) integrated within the controller/processor unit 36, or may be a separate controller. In some embodiments, illumination control unit 40 and/or controller/processor unit 36 may be part of telemetry unit 34.

The device 30 may further include a light sensing unit(s) 42 for sensing the light produced by the illuminating unit 38 and reflected from the walls of the GI tract. The light sensing unit(s) 42 may comprise a single light sensitive device or light sensor, or a plurality of discrete light sensitive device(s) or light sensor(s), such as but not limited to, a photodiode, a phototransistor, or the like. Other types of light sensors known in the art and having suitable characteristics may also be used for implementing the light sensing unit or units of embodiments of the present invention.

The light sensing unit(s) 42 may be suitably connected to the illumination control unit 40 for providing the illumination control unit 40 with a signal representative of the intensity of the light reflected from the walls of the gastrointestinal tract (or any other object within the field of view of the imaging unit 32). In operation, the illumination control unit 40 may process the signal received from the light sensing unit(s) 42 and, based on the processed signal, may control the operation of the light source(s) 38A, 38B, to 38N as is disclosed in detail hereinabove and hereinafter.

The device 30 may also include a power source 44 for providing power to the various components of the device 30. It is noted that for the sake of clarity of illustration, the connections between the power source 44 and the circuits or components of the device 30 which receive power therefrom, are not shown in detail. The power source 44 may be, for example, an internal power source similar to the power source(s) 25 of the device 10A, e.g., a battery or other power source. However, if the device 30 is configured as an insertable device (such as, for example, an endoscope-like device or a catheter -like device, or any other type of in vivo imaging device known in the art, the power source 44 may also be an external power source which may be placed outside the device 30 (such an external configuration is not shown in Fig. 2 for the sake of clarity of illustration). In such an embodiment having an external power source (not shown), the external power source (not shown) may be connected to the various power requiring components of the imaging device through suitable electrical conductors (not shown), such as insulated wires or the like.

It is noted that while for autonomous or swallowable in-vivo imaging device such as the device 10A the power source(s) 25 are preferably (but not necessarily) compact power sources for providing direct current (DC), external power sources may be any suitable power sources known in the art, including but not limited to power sources providing alternating current (AC) or direct current or may be power supplies coupled to the mains as is known in the art.

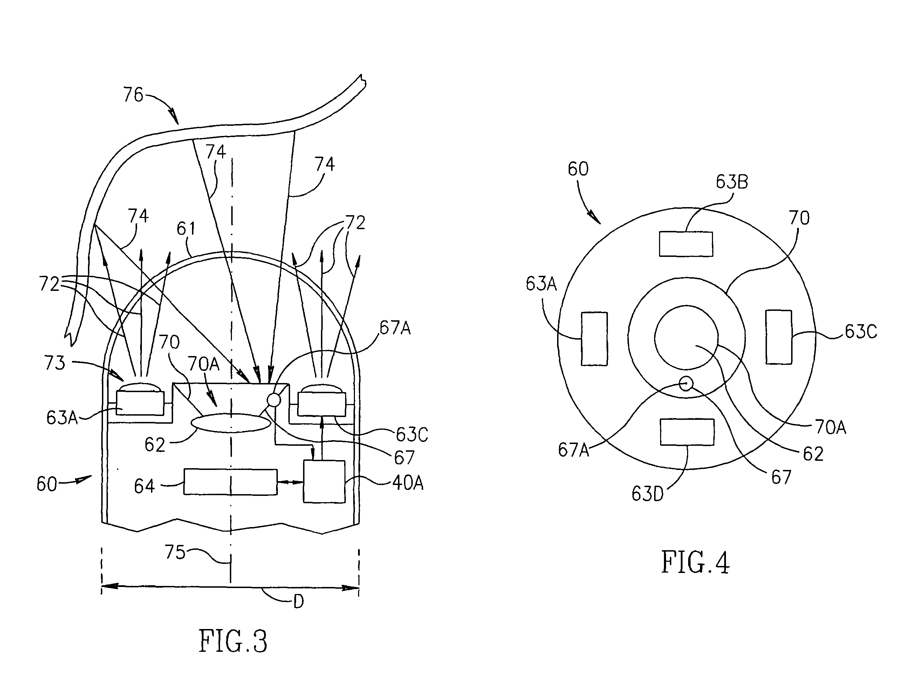

Reference is now made to Figs. 3 and 4. Fig. 3 is a schematic cross-sectional view of part of an in-vivo imaging device having an automatic illumination control system and four light sources, in accordance with an embodiment of the present invention. Fig. 4 is a schematic front view of the device illustrated in Fig. 3.

The device 60 (only part of which is shown in Fig. 3) includes an imaging unit 64. The imaging unit 64 may be similar to the imaging unit 32 of Fig. 2 or to the imaging unit 24 of Fig.1. Preferably, the imaging unit 64 may be a CMOS imaging unit, but other different types of imaging units may be also used. The imaging unit 64 may include CMOS imager circuitry, as is known in the art, but may also include other types of support and or control circuitry therein, as is known in the art and disclosed, for example, in U.S. patent 5,604,531 to Iddan et al., or in US 2001 035902 to Glukhovsky et al. The device 60 also includes an optical unit 62 which may comprise a lens or a plurality of optical elements as disclosed hereinabove for optical unit 22 of Fig. 1 and the optical unit 32A of Fig. 2.

The device 60 may include an illuminating unit 63, which may include four light sources 63A, 63B, 63C and 63D which may be disposed within the device 60 as shown in Fig. 4. The light sources 63A, 63B, 63C and 63D may be the white LED light sources or as disclosed, for example, in US 2001 035902 to Glukhovsky et al., but may also be any other suitable type of light sources, including but not limited to, infrared light sources, monochromatic light sources, band limited light sources known in the art or disclosed hereinabove.

It is noted that while in accordance with one embodiment of the present invention the light sources 63A, 63B, 63C and 63D are shown to be identical, other embodiments of the invention may be implemented with multiple light sources which may not be identical. Some of the light sources may have a spectral distribution, which is different from the spectral distribution of the other light sources. For example, of the light sources within the same device, one of the light sources may be a red LED, another light source may be a blue LED and another light source may be a yellow LED. Other configurations of light sources are also possible.

The device 60 may also include a baffle 70, which may be conically shaped or which may have any other suitable shape. The baffle 70 may have an aperture 70A therein. The baffle 70 may be interposed between the light sources 63A, 63B, 63C and 63D and the optical unit 62 and may reduce the amount of light coming directly from the light sources 63A, 63B, 63C and 63D to enter the aperture 70A. The device 60 may include a transparent optical dome 61 similar to the optical dome or window 21 of Fig. 1. The optical dome 61 may be made from a suitable transparent plastic material or glass or from any other suitable material which is sufficiently transparent to at least some of the wavelengths of light produced by the light sources 63A, 63B, 63C and 63D to allow for adequate imaging.

The device 60 may further include a light sensing unit 67 for sensing light, which is reflected from or diffused by the intestinal wall 76. The light sensing unit is attached to the baffle 70 such that its light sensitive part 67A faces the optical dome 61. Preferably, but not necessarily, the light sensing unit 67 may be positioned on the surface of baffle 70 at a position which allows the light sensing unit 67 to sense an amount of light which is representative or proportional to the amount of light entering the aperture 70A of the baffle 70. This may be true when the illuminated object is semi-diffusive (as the intestinal surface may be), and when the size of the light sensing unit 67 and its distance from the imaging sensor axis 75 are small compared to the diameter **D** of the capsule like device 60.

The device 60 (Fig. 3) is illustrated as being adjacent to the intestinal wall 76. In operation, light rays 72 which are generated by the light sources 63A, 63B, 63C and 63D may penetrate the optical dome 61 and may be reflected from the intestinal wall 76. Some of the reflected light rays 74 may pass the optical dome 61 and may reach the light sensing unit 67. Other reflected light rays (not shown) may reach the aperture 70A and pass the optical unit 62 to be focused on the imaging unit 64.

The amount of light measured by the light sensing unit 67 may be proportional to the amount of light entering the aperture 70A. Thus, the measurement of the light intensity reaching the light sensing unit 67 may be used to control the light output of the light sources 63A, 63B, 63C and 63D as is disclosed in detail hereinafter.

The device 60 also includes an illumination control unit 40A. The illumination control unit 40A is suitably coupled to the light sensing unit 67 and to the illuminating unit 63. The illumination control unit 40A may process the signal received from the light sensing unit 67 to control the light sources 63A, 63B, 63C and 63D as is disclosed in detail hereinafter.

The device 60 may also include a wireless transmitter unit (not shown in Fig. 3) and an antenna (not shown in Fig. 3), such as but not limited to the transmitter 26 and the antenna 27 of Fig. 1 or may include any suitable telemetry unit (such as, but not limited to the telemetry unit 34 of Fig. 2). The telemetry unit may be a transmitter or a transceiver, for wirelessly transmitting (and optionally also receiving) data and control signals to (and optionally from) an external receiver/recorder (not shown in Fig. 3) as disclosed in detail hereinabove. The device 60 may also include one or more power sources such as, for example, the power sources 25 of Fig. 1, or any other suitable power sources, known in the art.

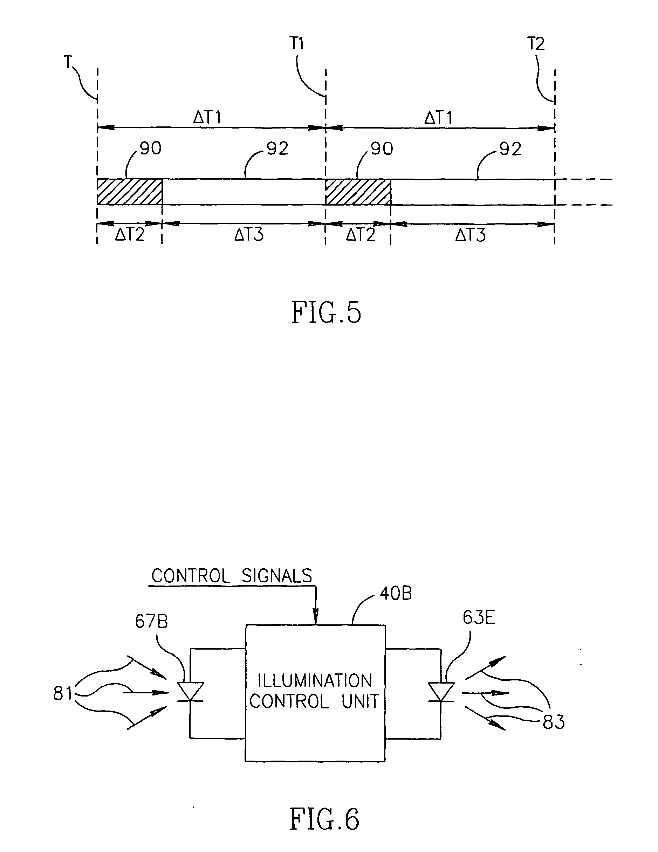

Reference is now made to Fig. 5 which is a schematic diagram illustrating a method of timing of the illumination and image acquisition in an in vivo imaging device having a fixed illumination duration. The timing method may be characteristic for imaging devices having CMOS imagers but may also be used in devices having other types of imagers.

An image acquisition cycle or period starts at the time T. The first image acquisition cycle ends at time T1 and has a duration $\Delta T1$. The second image acquisition cycle starts at time T1, ends at time T2 and has a duration $\Delta T1$. Each imaging cycle or period may comprise two parts, an illumination period 90 having a duration $\Delta T2$, and a dark period 92 having a duration $\Delta T3$. The illumination periods 90 are represented by the hatched bars of Fig. 5. During the illumination period 90 of each imaging cycle, the illumination unit (such as but not limited to the illuminating unit 38 of Fig. 2, or the illuminating unit 63 of Fig. 3) is turned on and provides light for illuminating the intestinal wall. During the dark period 92 of each imaging cycle, the illuminating unit (such as but not limited to the illuminating unit 38 of Fig. 2, or the illuminating unit 63 of Fig. 3) is switched off and does not provide light.

The dark period 92, or a part thereof, may be used for, for example, to acquiring an image from the imager by, for example, scanning the pixels of the imager and for processing the imager output signals and for transmitting the output signals or the processed output signals to an external receiver or receiver/recorder device, as disclosed hereinabove.

It is noted that while for the sake of simplicity, the diagram of Fig. 5 illustrates a case in which the image acquisition cycle duration is fixed, and imaging is performed at a fixed frame rate, this is not mandatory. Thus, the frame rate and therefore the image acquisition cycle duration may vary during imaging in accordance with a measured parameter such as, for example the velocity of the imaging device within the gastrointestinal tract.

Generally, different types of light control methods may be used for ensuring adequate image acquisition.

In a first method, the amount of light impinging on the light sensing unit 67 may be continuously measured and recorded during the illumination of the target tissue by the illuminating unit 63 to provide a cumulative value representative of the total cumulative number of photons detected by the light sensing unit 67. When this cumulative value reaches a certain value, the illuminating unit 63 may be shut off by switching off the light sources 63A, 63B, 63C, and 63D included in the illuminating unit 63. In this way the device 60 may ensure that when the quantity of measured light is sufficient to result in an adequately exposed frame (on the average), the illuminating unit 63 is turned off.

One advantage of the first method is that if the light sources (such as the light sources 63A, 63B, 63C, and 63D) are operated at their maximal or nearly maximal light output capacity, the switching off may save energy when compared to the energy expenditure in a fixed duration illumination period (such as the illumination period 90 of Fig. 5).

Another advantage of the first method is that it enables the shortening of the duration of the illumination period within the imaging cycle in comparison with using a fixed illumination period. In a moving imaging device, such as the device 60, ideally, it may be desirable to have the illumination period as short as practically possible, since this prevents or reduces image smearing due to the movement of the device 60 within the GI tract. Thus, typically, in a moving imaging device, the shorter the illumination period, the sharper will the resulting image be (assuming that enough light is generated by the illuminating unit to ensure adequate image exposure).

This may be somewhat similar to the increasing of the shutter speed in a regular shutter operated camera in order to decrease the duration of exposure to light to prevent smearing of the image of a moving object or image, except that in embodiments of the present method there is typically no shutter and the illumination period is being shortened controllably to reduce image smearing due to device movements in the GI tract.

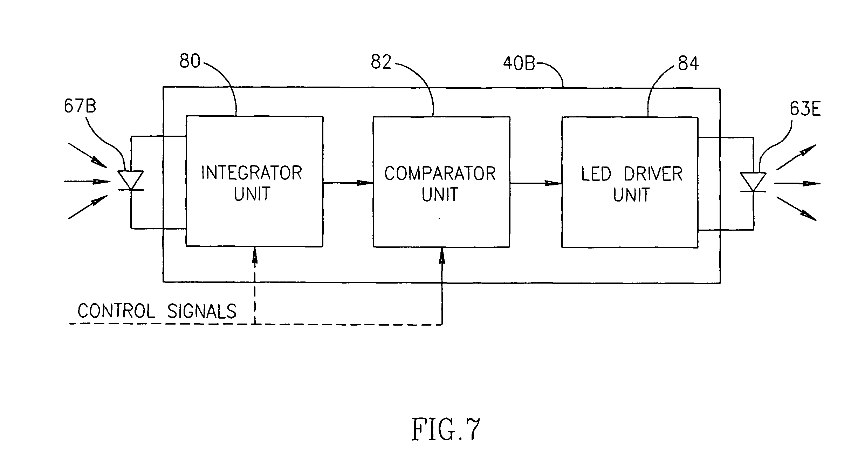

Reference is now made to Figs. 6 and 7. Fig. 6 is a schematic diagram illustrating one possible configuration for an illumination control unit coupled to a light sensing photodiode and to a light emitting diode, in accordance with an embodiment of the present invention. Fig. 7 is a schematic diagram illustrating the illumination control unit of Fig. 6 in detail, in accordance with an embodiment of the present invention.

The illumination control unit 40B of Fig. 6 may be suitably connected to a photodiode 67B, which may be operated as a light sensing unit. Any other suitable sensing unit or light sensor may be used. The illumination control unit 40B may be suitably connected to a light emitting diode (LED) 63E. The LED 63E may be a white LED as disclosed hereinabove or may be any other type of LED suitable for illuminating the imaged target (such as the gastrointestinal wall). The illumination control unit 40B may receive a current signal from the photodiode 67B. The received signal may be proportional to the intensity of light (represented schematically by the arrows 81) impinging the photodiode 67B. The illumination control 40B may process the received signal to determine the amount of light that illuminated the photodiode 67B within the duration of a light measuring time period. The illumination control 40B may control the energizing of the LED 63E based on the amount of light that illuminated the photodiode 67B within the duration of the light measuring time period. Examples of the type of processing and control of energizing are disclosed in detail hereinafter. The illumination control unit 40B may also receive control signals from other circuitry components included in the in vivo imaging device. For example, the control signals may include timing and/or synchronization signals, on/off switching signals, reset signals, or the like.

The light sensing unit(s) and light producing unit(s) may be any suitable light producing or sensing units other than diodes.

Fig. 7 illustrates one possible embodiment of the illumination control unit 40B. The illumination control unit 40B may include, for example, an integrator unit 80, a comparator unit 82 and an LED driver unit 84. The integrator unit 80 is coupled to the photodiode 67B to receive therefrom a signal indicative of the intensity of the light impinging on the photodiode 67B, and to record and sum the amount of light impinging on the photodiode 67B. The integrator unit 80 may be suitably connected to the comparator unit 82.

The integrator unit 80 may record and sum the amount of light impinging on the photodiode 67B, integrating the received signal, and output an integrated signal to the comparator unit 82. The integrated signal may be proportional to or indicative of the cumulative number of photons hitting the photodiode 67B over the integration time period. The comparator unit 80 may be suitably connected to the LED driver unit 84. The comparator unit 80 may continuously compare the value of the integrated signal to a preset threshold value. When the value of the integrated signal is equal to the threshold value, the comparator unit 82 may control the LED driver unit 84 to switch off the power to the LED 63E and thus cease the operation of the LED 63E.

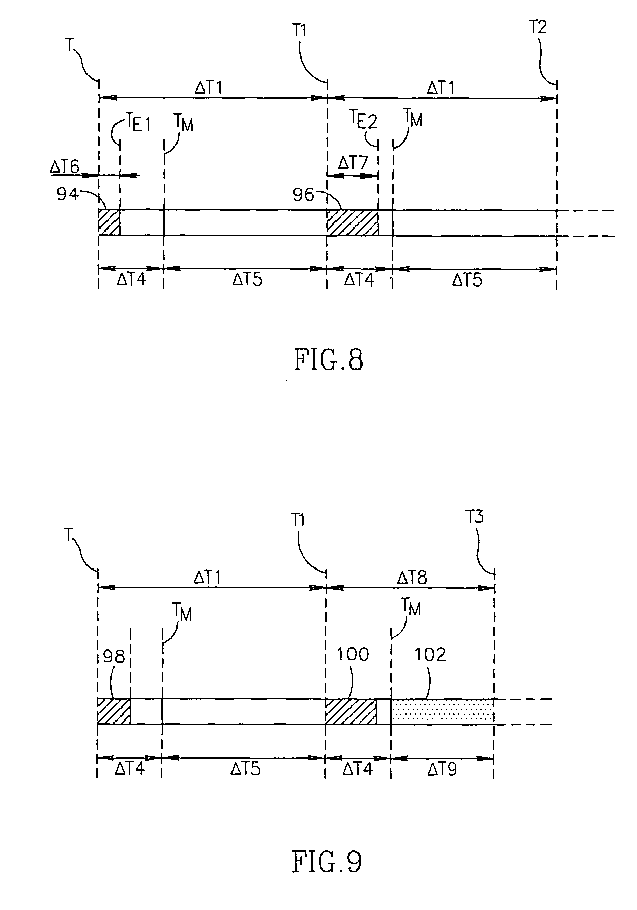

Thus, the illumination control unit 40A may be constructed and operated similar to the illumination control unit 40B of Figs. 7 and 8.

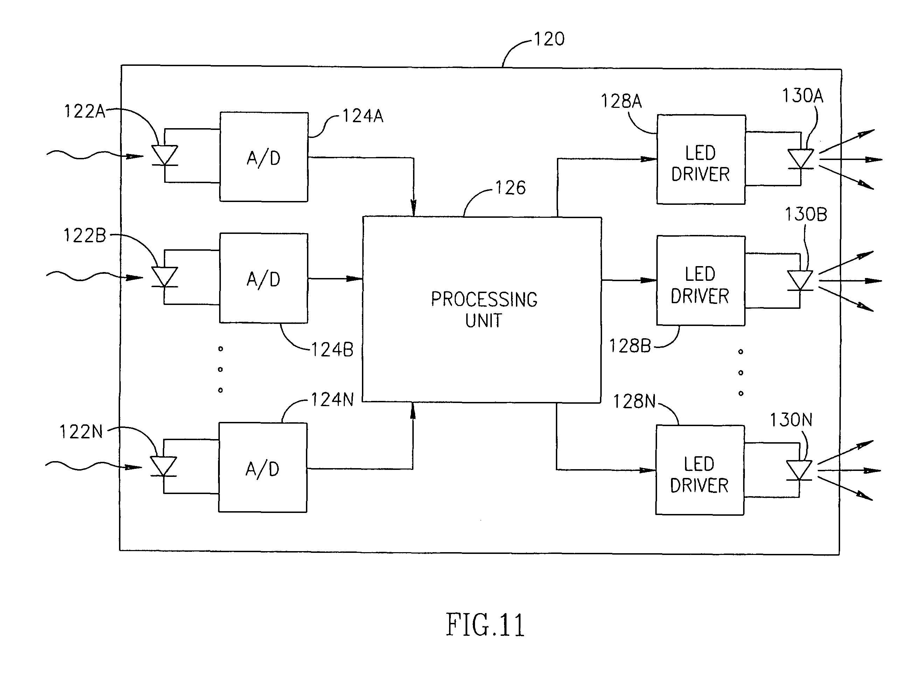

It is noted that while the circuits illustrated in Fig. 7 may be implemented as analog circuits, digital circuits and/or hybrid analog/digital circuits may be used in implementing the illumination control unit, as is disclosed in detail hereinafter (with respect to Fig. 11).

Reference is now made to Fig. 8, which is a schematic diagram useful for understanding a method of timing of the illumination and image acquisition in an in vivo imaging device having a variable controlled illumination duration, according to one embodiment.

An image acquisition cycle or period starts at the time T. The first image acquisition cycle ends at time T1 and has a duration $\Delta T1$. The second image acquisition cycle starts at time T1, ends at time T2 and has a duration $\Delta T1$. In each imaging cycle, the time period having a duration $\Delta T4$ defines the maximal allowable illumination period. The maximal allowable illumination period $\Delta T4$ may typically be a time period which is short enough as to enable imaging without excessive image smearing or blurring due to the movement of the device 60 within the GI tract. The time $T_M$ is the time of the end of the maximal allowable illumination period $\Delta T4$ relative to the beginning time of the first imaging cycle.

The maximal allowable illumination period $\Delta T4$ may be factory preset taking into account, inter alia, the typical or average (or maximal) velocity reached by the imaging device within the GI tract, (as may be determined empirically in a plurality of devices used in different patients), the type of the imaging sensor (such as, for example, the CMOS sensor 64 of the device 50) and its scanning time requirements, and other manufacturing and timing considerations. In accordance with one implementation of the invention, when imaging at 2 frames per second $\Delta T1 = 0.5$ second, the duration of $\Delta T4$ may be set to have a value in the range of 20-30 milliseconds. However, this duration is given by way of example only, and $\Delta T4$ may have other different values. Typically, the use of a maximal allowable illumination period $\Delta T4$ of less than 30 millisecond may result in acceptable image quality of most of the acquired image frames without excessive degradation due to blurring of the image resulting from movement of the imaging device within the GI tract.

The time period $\Delta T5$ is defined as the difference between the entire imaging cycle duration $\Delta T1$ and the maximal allowable illumination period $\Delta T4$ ($\Delta T5 = \Delta T1 - \Delta T4$).

At the time of beginning T of the first imaging cycle, the illumination unit (such as but not limited to the illuminating unit 63 of Fig. 3) is turned on and provides light for illuminating the intestinal wall. The light sensing unit 67 senses the light reflected and/or diffused from the intestinal wall 76 and provides a signal to the illumination control unit 40A of the device 60. The signal may be proportional to the average amount of light entering the aperture 70A. The signal provided by the light sensing unit 67 may be integrated by the illumination control unit 40A as is disclosed in detail hereinabove with respect to the illumination control unit 40B of Figs. 7 and 8.

The integrated signal may be compared to a preset threshold value (for example by a comparator such as the comparator unit 82 of Fig. 8). When the integrated signal is equal to the threshold value, the illumination control unit 40A ceases the operation of the light sources 63A, 63B, 63C and 63D of the illuminating unit 63. The time $T_{E1}$ is the time at which the illuminating control unit turns off the light sources 63A, 63B, 63C and 63D within the first imaging cycle. The time interval beginning at time T and ending at time $T_{E1}$ is the illumination period 94 (represented by the hatched bar labeled 94) for the first imaging cycle. The illumination period 94 has a duration $\Delta T6$. It may be seen that for the first imaging cycle $\Delta T6 < \Delta T4$.

After the time $T_{E1}$ the scanning of the pixels CMOS sensor 64 may begin and the pixel data (and possibly other data) may be transmitted by the transmitter (not shown in Fig. 3) or telemetry unit of the device 60.

Preferably, the scanning of the pixels of the CMOS sensor 64 may begin as early as the time $T_{E1}$ of the termination of the illumination. For example the illumination control unit 40A may send a control signal to the CMOS sensor at time $T_{E1}$ to initiate the scanning of the pixels of the CMOS sensor 64. However, the scanning of the pixels may also begin at a preset time after the time $T_M$ which is the ending time of the maximal allowable illumination period $\Delta T4$, provided that sufficient time is available for pixel scanning and data transmission operations.

At the time of beginning T1 of the second imaging cycle, the illuminating unit 63 is turned on again. The light sensing unit 67 senses the light reflected and/or diffused from the intestinal wall 76 and provides a signal to the illumination control unit 40A of the device 60. The signal may be proportional to the average amount of light entering the aperture 70A.

The signal provided by the light sensing unit 67 may be integrated and compared to the threshold value as disclosed hereinabove for the first imaging cycle. When the integrated signal is equal to the threshold value, the illumination control unit 40A turns off the light sources 63A, 63B, 63C and 63D of the illuminating unit 63. However, in the particular schematic example illustrated in Fig. 8, the intensity of light reaching the light sensing unit 67 in the second imaging cycle is lower than the intensity of light reaching the light sensing unit 67 in the first imaging cycle.

This difference of the illumination intensity or intensity versus time profile between different imaging cycle may be due to, inter alia, movement of the device 60 away from the intestinal wall 76, or a change of the position or orientation of the device 60 with respect to the intestinal wall 76, or a change in the light absorption or light reflecting or light diffusion properties of the part of the intestinal wall 76 which is within the field of view of the device 60.

Therefore it takes longer for the integrated signal output of the integrator unit to reach the threshold value. Therefore, the illumination control unit 40A turns the illuminating unit 63 off at a time $T_{E2}$ (it is noted that $T_{E2} > T_{E1}$).

The time interval beginning at time T1 and ending at time $T_{E2}$ is the illumination period 96 for the second imaging cycle. The illumination period 96 (represented by the hatched bar labeled 96) has a duration $\Delta T7$. It may be seen that for the second imaging cycle $\Delta T7 < \Delta T4$.

Thus, the duration of the illumination period within different imaging cycles may vary and may depend, inter alia, on the intensity of light reaching the light sensing unit 67.

After the time $T_{E2}$ the scanning of the pixels CMOS sensor 64 may begin and the pixel data (and possibly other data) may be transmitted as disclosed in detail hereinabove for the first imaging cycle of Fig. 8.

It is noted that while for the sake of simplicity, the diagram of Fig. 8 illustrates a case in which the image acquisition cycle duration $\Delta T1$ is fixed, and imaging is performed at a fixed frame rate, this is not mandatory. Thus, the frame rate and therefore the image acquisition cycle duration $\Delta t1$ may vary during imaging in accordance with a measured parameter such as, for example the velocity of the imaging device within the gastrointestinal tract. In such cases, the duration of the imaging cycle may be shortened or increased in response to the measured velocity of the device 60 in order to increase or decrease the frame rate, respectively.

For example, US 6,709,387 assigned to the assignee of the present application, incorporated herein by reference in its entirety for all purposes, discloses, inter alia, a device and method for controlling the frame rate of an in-vivo imaging device.

The automatic illumination control methods disclosed hereinabove may be adapted for use in a device having variable frame rate. Such adaptation may take into account the varying duration of the imaging cycle and the implementation may depend, inter alia, on the amount of time required to complete the pixel scanning and the data transmission, the available amount of power available to the device 60, and other considerations.

A simple way of adapting the method may be to limit the maximal frame rate of the imaging device, such that even when the maximal frame rate is being used, there will be enough time left for pixel scanning and data transmission within the time period.

Reference is now made to Fig. 9, which is a schematic diagram useful for understanding a method of timing of the illumination and image acquisition in an in vivo imaging device having a variable frame rate and a variable controlled illumination duration.

The first imaging cycle of Fig. 9 is similar to the first imaging cycle of Fig. 8 except that the duration of the illumination period 98 of Fig. 9 (represented by the hatched bar labeled 98) is longer than the duration of the illumination period 94 of Fig. 8. The first imaging cycle of Fig. 9 starts at time T, ends at time T1, and has a duration $\Delta T1$. The time $T_M$ represents the end of the maximal allowable illumination period $\Delta T4$. The second imaging cycle of Fig. 9 begins at time T1 and ends at time T3. The duration of the second imaging cycle $\Delta T8$ is shorter than the duration of the first imaging cycle $\Delta T1$ ($\Delta T8 < \Delta T1$). The duration of the second imaging cycle $\Delta T8$ corresponds with the highest frame rate usable in the imaging device. The illumination period 100 of the second imaging cycle (represented by the hatched bar labeled 100 of Fig. 9) is timed by the illumination control unit depending on the light intensity as disclosed in detail hereinabove. The time period 102 (represented by the dotted bar labeled 102) represents the amount of time $\Delta T9$ required for scanning the pixels of the imager and transmitting the scanned frame data. $T_M$ represents the time of ending of the maximal allowable illumination period relative to the beginning time of each imaging cycle. Thus, if the frame rate is increased, even at the highest possible frame rate there is enough time to scan the pixels and transmit the data.

It is noted that typically, in an exemplary in vivo imaging device having a fixed frame rate, the time required for scanning the pixels of a CMOS sensor having 64,000 pixels (such as but not limited to a CMOS sensor arranged in a 256 X 256 pixel array), and for transmitting the analog data signals to an external receiver recorder may be approximately 0.4 milliseconds (assuming a scanning and data transmission time of approximately 6 microseconds per pixel). Thus, assuming a maximal illumination period of approximately 20-30 milliseconds, the frame rate may not be extended much higher than 2 frames per second. Alternate frame rates may be used.

It may however be possible to substantially shorten the time required for scanning the pixels and for transmitting the data. For example, by increasing the clock rate of the CMOS pixel array, it may be possible to reduce the time required to scan an individual pixel to 3 microseconds or even less. Additionally, it may be possible to increase the data transmission rate of the transmitter 26 to even further shorten the overall time required for scanning the array pixels for transmitting the pixel data to the external receiver/recorder.

Therefore, variable frame rate in vivo imaging devices, as well as fixed frame rate devices, may be implemented which may be capable of frame rates of approximately 4-8 frames per second, and even higher.

When the method disclosed hereinabove for turning off the illuminating unit when the integrated output of the light sensing unit reaches a threshold value adapted to ensure a good average image quality is implemented, the tendency of the designer would be to operate the illuminating unit (such as, for example the illuminating unit 63 of Fig. 3) close to the maximal available light output capacity. This may be advantageous because of the shortened illumination period duration achievable which may improve image clarity by reducing movement induced image blurring.

It may not always be possible or desired to operate the illuminating unit close to the maximal possible light output capacity. Therefore, it may be desired to start the operation of the illuminating unit 63 at a given light output which is lower than the maximal light output of illuminating unit 63.

In a second illumination control method, the illuminating unit 63 of Fig. 3 may be initially operated at a first light output level at the beginning of each of the imaging cycles. The light sensing unit 67 may be used to measure the amount of light during a short illumination sampling period.

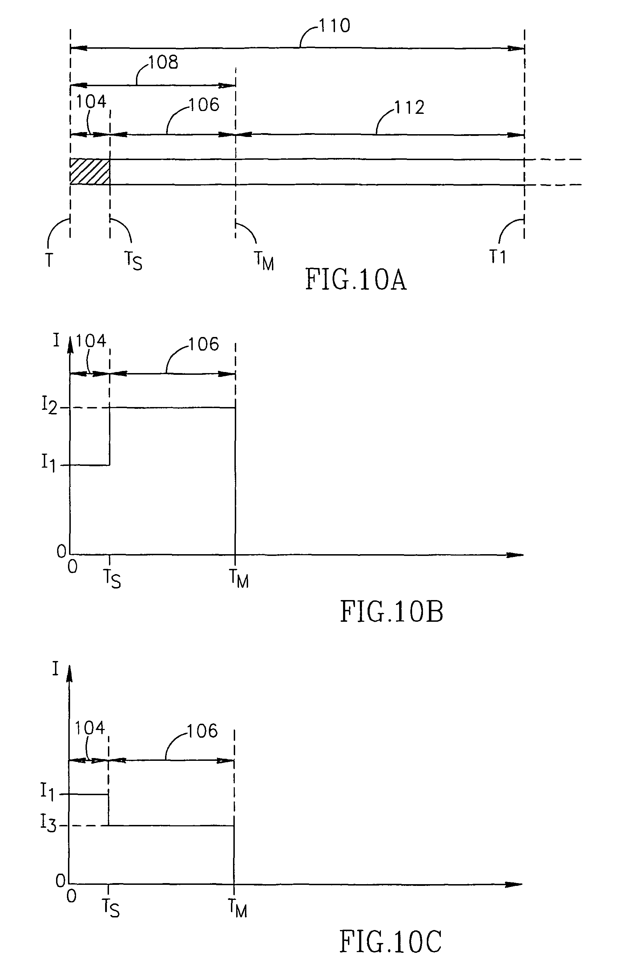

Reference is now made to Figs. 10A, 10B and 10C. Fig 10A is a timing diagram schematically illustrating an imaging cycle of an in vivo imaging device using an automatic illumination control method in accordance with another embodiment of the present invention. Fig. 10B is an exemplary schematic graph representing an example of the light intensity as a function of time, possible when using the method of automatic illumination control illustrated in Fig. 10A. Fig. 10C is a schematic graph representing another example of the light intensity as a function of time, possible when using the method of automatic illumination control illustrated in Fig. 10A.

In Figs. 10A, 10B and 10C, the horizontal axes of the graphs represents time in arbitrary units. In Figs. 10B and 10C, the vertical axis represents the intensity I of the light output by the illuminating unit 63 (Fig. 3).

The automatic illumination control method illustrated in Fig. 10A operates by using an illumination sampling period 104 included in a total illumination period 108. An imaging cycle 110 includes the total illumination period 108 and a dark period 112. The illuminating unit 63 may illuminate the intestinal wall 76 within the duration total illumination period 108. The dark period 112 may be used for scanning the pixels of the CMOS imager 64 and for processing and transmitting the image data as disclosed in detail hereinabove.

The total illumination period of the imaging cycle starts at time T and ends at time $T_M$. The time $T_M$ is fixed with respect to the beginning time T of the imaging cycle 110, and represents the maximal allowable illumination time. Practically, the time $T_M$ may be selected to reduce the possibility of image blurring as explained hereinabove. For example, the time $T_M$ may be selected as 20 milliseconds from the time of beginning T of the imaging cycle 110 (in other words, the duration of the total illumination period 108 may be set at 30 milliseconds), but other larger or smaller values of the time $T_M$ and of the total illumination period 108 may also be used.

The total illumination period 108 may include an illumination sampling period 104 and a main illumination period 106. The illumination sampling period 104 starts at time T and ends at time $T_S$. The main illumination period 106 starts at time $T_S$ and ends at time $T_M$.

In an exemplary embodiment of the method, the duration of the illumination sampling period 104 may be set at approximately 2-5 milliseconds, but other larger or smaller duration values may be used depending, inter alia, on the type and characteristics of the light sensing unit 67, its sensitivity to light, its signal to noise ratio (S/N), the intensity $I_1$ at which the illuminating unit 63 is operated during the illumination sampling period 104, and other implementation and manufacturing considerations.

Turning to Figs. 10B and 10C, during the illumination sampling period 104, the illuminating unit 63 is operated such that the intensity of light is $I_1$. The light sensing 67 may sense the light reflected from and diffused by the intestinal wall 76. The illumination control unit 40A may integrate the intensity signal to determine the quantity **Q** of light reaching the light sensing unit 67 within the duration of the illumination sampling period 104. The illumination control unit 40A may then compute from the value **Q** and from the known duration of the main illumination period 106, the intensity of light $I_N$ at which the illuminating unit 63 needs to be operated for the duration of the main illumination period 106 in order to provide adequate average exposure of the CMOS sensor 64. In one embodiment an estimated total amount of light received is kept substantially constant across a set of imaging cycles, or is kept within a certain target range. The computation may be performed, for example, by subtracting from a fixed light quantity which is desired to be received or applied the amount of light recorded during the sampling period 104 and dividing the result by a fixed time period which corresponds to the main illumination period 106. One possible way to perform the computation would be using equation 1 as follows:

$$I_N = (Q_T - Q)/\Delta T_{MAIN} \qquad\qquad \text{equation 1}$$

Wherein,

$\Delta T_{MAIN}$ is the duration of the main illumination period 106,
$Q_T$ is the total quantity of light that needs to reach the light sensing unit 67 within an imaging cycle to ensure adequate average exposure of the CMOS sensor 64, and
**Q** is the quantity of light reaching the light sensing unit 67 within the duration of an illumination sampling period 104 of an imaging cycle.

It is noted that the value of $Q_T$ may be empirically determined.

Figs. 10B schematically illustrates a graph showing the intensity of light produced by the illuminating unit 63 as a function of time for an exemplary imaging cycle. During the illumination sampling period 104 the light intensity has a value $I_1$. After the end of the illumination sampling period 104, the light intensity $I_N = I_2$ may be computed as disclosed in equation 1 hereinabove, or by using any other suitable type of analog or digital computation.

For example, if the computation is digitally performed by the controller/processor 36 of Fig. 2, the value of $I_N$ may be computed within a very short time (such as for example less than a microsecond) compared to the duration of the main illumination period 106.

If the computation of $I_N$ is performed by an analog circuit (not shown) which may be included in the illumination control unit 40 of Fig. 2, or in the illumination control unit 40B of Fig. 6, or in the illumination control unit 40A of Fig. 3, the computation time may also be short compared to the duration of the main illumination period 106.

After the computation of $I_2$ for the imaging cycle represented in Fig. 10B is completed, the illumination control unit 40A may change the intensity of the light output of the illuminating unit of the imaging device to $I_2$. This may be achieved, for example, by increasing the amount of current output from the LED driver unit 84 of Fig. 7, or by increasing the amount of current output from one or more LED driver units (not shown in detail) which may be included in the illumination control unit 40A to supply current to the light sources 63A, 63B, 63C, and 63D. At the end of the main illumination period 108 (at time TM), the illumination control unit 40A may switch the illuminating unit 63 off until time T1 which is the beginning of a new imaging cycle (not shown). At the beginning of the new imaging cycle, the light intensity is switched again to the value $I_1$ and a new illumination sampling period begins.

Fig. 10C schematically illustrates a graph showing the intensity of light produced by the illuminating unit 63 as a function of time for another different exemplary imaging cycle. The illumination intensity $I_1$ is used throughout the illumination sampling period 104 as disclosed hereinabove. In this imaging cycle, however, the value of **Q** measured for the illumination sampling period 104 is higher than the value of **Q** measured for the illumination sampling period of Fig.

10B. This may happen, for example, due to movement of the position of the imaging device 60 relative to the intestinal wall 76. Therefore the computed value of $I_3$ is lower than the value of $I_2$ of the imaging cycle illustrated in Fig. 10B. The value of $I_3$ is also lower than the value of $I_1$. Thus, the intensity of light emitted by the illuminating unit 63 during the main illuminating period 106 illustrated in Fig. 10C is lower than the intensity of light emitted by the illuminating unit 63 during the illumination sampling period 104 of Fig. 10C.

It is noted that if the computed value of $I_3$ is equal to the value of $I_1$ (case not shown in Figs. 10B-10C) the illumination intensity may be maintained at the initial value of $I_1$ for the duration of the total illumination period 108, and no modification of the illumination intensity is performed at time $T_M$.

An advantage of the second illumination control method disclosed hereinabove may be that it may at least initially avoid the operating of the illuminating unit 63 at its maximal light output intensity. This may be useful for improving the performance of the power sources, such as, for example, the power source(s) 25 of Fig. 1, and may extend the useful operational life thereof. It is known in the art that many batteries and electrochemical cells do not perform optimally when they are operated near their maximal current output. When using the second illumination method, the light sources (such as the light sources 63A, 63B, 63C, and 63D of Fig. 3) are initially operated at a light intensity $I_1$ which may be a fraction of their maximal output light intensity. Thus, in cases where it is determined that the maximal light output intensity is not required for the current frame acquisition, the light sources may be operated at a second light intensity level (such as, for example the light intensity level $I_3$ which is lower than the light intensity level $I_1$). Thus, the second illumination control method may reduce the current required for operating the illuminating unit 63 drawn from the batteries or other power sources of the imaging device which may extend the useful operational life of the batteries or of other power sources used in the imaging device.

It will be appreciated by those skilled in the art that the embodiments of the present invention are not limited to the use of a single light sensing element and/or a single light source.

Reference is now made to Fig. 11 which is a schematic diagram illustrating an illumination control unit including a plurality of light sensing units for controlling a plurality of light sources, in accordance with an embodiment of the present invention.

The illumination control unit 120 includes a plurality of light sensing units 122A, 122B,....122N, suitably interfaced with a plurality of analog to digital (A/D) converting units 124A, 124B,...124N, respectively. The A/D converting units are suitably connected to a processing unit 126. The processing unit 126 is suitably connected to a plurality of LED drivers 128A, 128B,...128N which are suitably connected to a plurality of LED light sources 130A, 130B,...130N.

Signals representing the intensity of light sensed by the light sensing units 122A, 122B,....122N are fed to the A/D converting units 124A, 124B,...124N, respectively, which output digitized signals. The digitized signals may be received by the processing unit 126 which may process the signals. For example the processing unit 136 may perform integration of the signals to compute the quantity of light sensed by the light sensing units 122A, 122B,....122N. The computed quantity of light may be the total combined quantity of light sensed by all the light sensing units 122A, 122B,....122N taken together, or may be the individual quantities of light separately computed for each individual light sensing unit of the light sensing units 122A, 122B,....122N.

The processing unit 136 may further process the computed light quantity or light quantities, to provide control signals to the LED drivers 128A, 128B,...128N which in turn provide the suitable currents to the LED light sources 130A, 130B,...130N.

It is noted that the illumination control unit 120 of Fig. 11 may be operated using different processing and control methods.

In accordance with one embodiment of the present invention, all the light sensing units 122A, 122B,....122N may be used as a single light sensing element and the computation is performed using the combined total quantity of light to simultaneously control the operation of all the LED light sources 130A, 130B,...130N together. In this embodiment, the illumination control unit 120 may be implemented using the first illumination control method as disclosed hereinabove and illustrated in Figs. 5, 8, and 9, which use a fixed illumination intensity and computes the termination time of the illumination.

Alternatively, in accordance with another embodiment of the present invention, the illumination control unit 120 may be implemented using the second illumination control method as disclosed hereinabove and illustrated in Figs. 10A-10C which uses a first illumination intensity $I_1$ in an illumination sampling period and computes a second light intensity $I_N$ for use in a main illumination period as disclosed in detail hereinabove. In such a case, the illumination intensity $I_1$ used throughout the illumination sampling period 104 (see Figs. 10A-10C) may be identical for all the LED light sources 130A, 130B,...130N, and the illumination intensity $I_N$ used throughout the main illumination period 106 (Figs. 10A-10C) may be identical for all the LED light sources 130A, 130B,...130N.

In accordance with another embodiment of the present invention, each of the light sensing units 122A, 122B,....122N may be used as a separate light sensing unit and the computation may be performed using the individual quantities of light sensed by each of the light sensing units 122A, 122B,....122N to differentially control the operation of each of the LED light sources 130A, 130B, ...130N separately. In this second embodiment, the illumination control unit 120 may be implemented using the first illumination control method as disclosed hereinabove and illustrated in Figs. 5, 8, and 9, which uses a fixed illumination intensity for each of the LED light sources 130A, 130B,...130N and may separately compute the termination time of the illumination for each of the LED light sources 130A, 130B,...130N. In such a manner, sets of light sources 130A, 130B,...130N (where a set may include one) may be paired with sets of sensors 122A, 122B,....122N.

Alternatively, in accordance with another embodiment of the present invention, the illumination control unit 120 may be implemented using the second illumination control method as disclosed hereinabove and illustrated in Figs. 10A-10C which uses a first illumination intensity $I_1$ in an illumination sampling period and computes a second light intensity $I_N$ for use in a main illumination period as disclosed in detail hereinabove. In such a case, the illumination intensity $I_1$ may be identical for all the LED light sources 130A, 130B,...130N, and the illumination intensity $I_N$ may be identical for all the LED light sources 130A, 130B,...130N.

Typically, this embodiment may be used in cases in which the positioning of the light sources 130A, 130B, ... 130N and the light sensing units 122A, 122B,....122N in the imaging device are configured to ensure that a reasonably efficient "local control" of illumination is enabled and that the cross-talk between different light sources is at a sufficiently low level to allow reasonable local control of the illumination intensity produced by a one or more of the light sources 130A, 130B,...130N by processing the signals from one or more light sensing unit which are associated in a control loop with the one or more light sources.

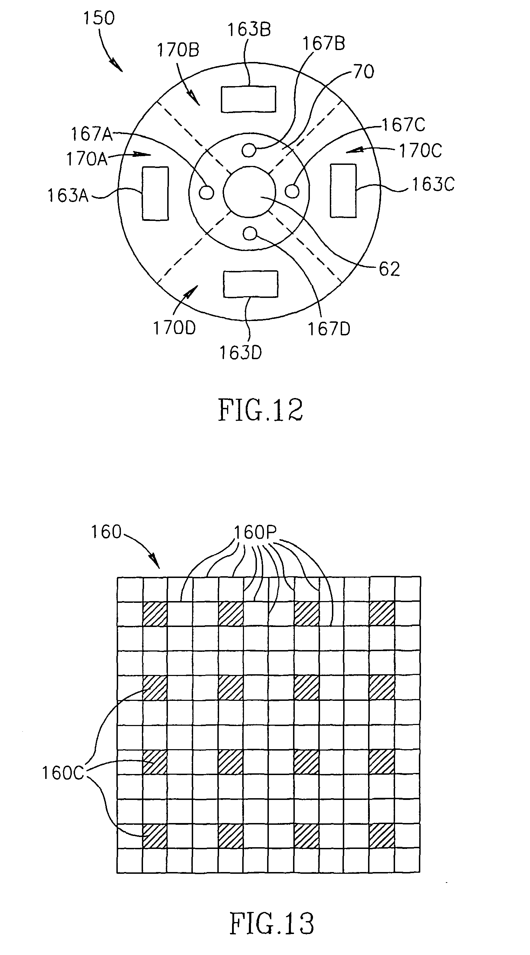

Reference is now made to Fig. 12 which is a schematic diagram illustrating a front view of an autonomous imaging device having four light sensing units and four light sources, in accordance with an embodiment of the present invention.

The device 150 includes four light sources 163A, 163B, 163C and 163D and four light sensing units 167A, 167B, 167C and 167D. The light sources 163A, 163B, 163C and 163D may be the white LED sources as disclosed hereinabove, or may be other suitable light sources. The light sensing units 167A, 167B, 167C and 167D are attached on the surface of the baffle 70, surrounding the aperture 62. The front part of the device 150 may include four quadrants 170A, 170B, 170C and 170D. The device 150 may include an illumination control unit (not shown in the front view of Fig. 12), and all the optical components, imaging components, electrical circuitry, and power source(s) for image processing and transmitting as disclosed in detail hereinabove and illustrated in the drawing Figures (See Figs. 1, 2).

The quadrants are schematically represented by the areas 170A, 170B, 170C and 170D between the dashed lines. In accordance with an embodiment of the invention, the device 150 may include four independent local control loops. For example, the light source 163A and the light sensing unit 167A which are positioned within the quadrant 170A may be suitably coupled to the illumination control unit (not shown) in a way similar to the coupling of the light sources 38A-38N and the light sensing unit(s) 42 to the illumination control unit 40 of Fig. 2. The signal from the light sensing unit 167A may be used to control the illumination parameters of the light source 163A using any of the illumination control methods disclosed hereinabove, forming a local control loop for the quadrant 170A.

Similarly, the signal from the light sensing unit 167B may be used to control the illumination parameters of the light source 163B using any of the illumination control methods disclosed hereinabove, forming a local control loop for the quadrant 170B, the signal from the light sensing unit 167C may be used to control the illumination parameters of the light source 163C using any of the illumination control methods disclosed hereinabove, forming a local control loop for the quadrant 170C, and the signal from the light sensing unit 167D may be used to control the illumination parameters of the light source 163D using any of the illumination control methods disclosed hereinabove, forming a local control loop for the quadrant 170D.

It is noted that there may be some cross-talk or interdependency between the different local control loops, since practically, some of the light produced by the light source 163A may be reflected from or diffused by the intestinal wall and may reach the light sensing units 167B, 167C, and 167D which form part of the other local control loops for the other quadrants 170B, 170C, and 170D, respectively.

The arrangement of the positions light sensing units 167A, 167B, 167C and 167D and the light sources 163A, 163B, 163C and 163D within the device 150 may be designed to reduce such cross-talk.

In other embodiments of the invention it may be possible to use processing methods such as "fuzzy logic" methods or neural network implementations to link the operation of the different local control loops together. In such implementations, the different local control loops may be coupled together such that information from one of the light sensing unit may influence the control of illumination intensity of light sources in other local control loops.

It is noted that, while the imaging device 150 illustrated in Fig. 12 includes four light sources and four light sensing units, The number of light sources may vary and the imaging device of embodiments of the present invention may be constructed with a different number (higher or lower than four) of light sources. Similarly, the number of the light sensing units may also vary, and any suitable or practical number of light sensing units may be used. Additionally, it is noted that the number of light sensing units in a device need not be identical to the number of light sources included in the device. Thus, for example, a device may be constructed having three light sensing units and six light sources. Or in another example, a device may be constructed having ten light sensing units and nine light sources.

The factors determining the number of light sources and the number of light sensing units may include, inter alia, the geometrical (two dimensional and three dimensional) arrangement of the light sources and the light sensing units within the device and their arrangement relative to each other, the size and available power of the light sources, the size and sensitivity of the light sensing units, manufacturing and wiring considerations.

The number of local control loops may also be determined, inter alia, by the degree of uniformity of illumination desired, the degree of cross-talk between the different local control loops, the processing power of the illumination control unit available, and other manufacturing considerations.

The inventors of the present invention have noticed that it is also possible to achieve illumination control using one or more of the light sensitive pixels of the imager itself, instead of or in addition to using dedicated light sensing unit (s) which are not part of the imager. In addition, it may be possible to use special light sensing elements integrated into the pixel array on the surface of the CMOS imager IC.

For example, in imagers having a CMOS type imager, some of the pixels of the CMOS imager may be used for controlling the illumination, or alternatively, specially manufactured light sensitive elements (such as, analog photodiodes, or the like) may be formed within the pixel array of the imager.

Reference is now made to Fig. 13 which is a top view schematically illustrating the arrangement of pixels on the surface of a CMOS imager usable for illumination control, in accordance with an embodiment of the present invention. It is noted that the pixel arrangement in Fig. 13 is only schematically illustrated and the actual physical arrangement of the circuitry on the imager is not shown.

The surface of the CMOS imager 160 is schematically represented by a 12X12 array comprising 144 square pixels. The regular pixels 160P are schematically represented by the white squares. The CMOS imager also includes sixteen control pixels 160C, which are schematically represented by the hatched squares.

It is noted that while the number of the pixels in the CMOS imager 160 was arbitrarily chosen as 144 for the sake of simplicity and clarity of illustration only, the number of pixels may be larger or smaller if desired. Typically, a larger numbers of pixels may be used to provide adequate image resolution. For example a 256X256 pixel array may be suitable for GI tract imaging.

In accordance with an embodiment of the present invention, the control pixels 160C may be regular CMOS imager pixels which are assigned to be operated as control pixels. In accordance with this embodiment, the control pixels 160C may be scanned at a different time than the regular imaging pixels 160P. This embodiment has the advantage that it may be implemented with a regular CMOS pixel array imager.

Turning back to Fig. 10A, the timing diagram of Fig. 10A may also be used to illustrate the automatic illumination control method using control pixels. The method may operate by using a fast scan of the control pixels 160C at the beginning of each imaging cycle 110. The illuminating unit (not shown) may be turned on at the beginning of the imaging cycle 110 (at time T). The scanning of the control pixels 160C may be performed similar to the scanning of the regular pixels 160P, except that the scanning of all of the control pixels 160C occurs within the illumination sampling period 104. The control pixels 160C may be serially scanned within the duration of the illumination sampling period 104. This is possible due to the ability to randomly scan any desired pixel in a CMOS pixel array, by suitably addressing the pixel readout lines (not shown) as is known in the art.

It is noted that since the control pixels 160C are scanned serially (one after the other), the control pixel which is scanned first has been exposed to light for a shorter time period than the control pixels which are scanned next. Thus, each control pixel is scanned after it has been exposed to light for a different exposure time period.

If one assumes that the intensity of light reflected from the intestinal wall does not change significantly within the duration of the illumination sampling period 104, it may be possible to compensate for this incrementally increasing pixel exposure time by computationally correcting the average measured light intensity for all the control pixels 160C, or the computed average quantity of light reaching all the control pixels 160C. For example, a weighted average of the pixel intensities may be computed.

Alternatively, in accordance with another embodiment of the present invention, the illuminating unit 63 may be turned off after the end of the illumination sampling period 104 (the turning off is not shown in Fig. 10A). This turning off may enable the scanning of the control pixels 160C while the pixels 160C are not exposed to light and may thus prevent the above described incremental light exposure of the control pixels.

After the scanning (readout) of all the control pixels 160C is completed and the scanned control pixel signal values are processed (by analog or by digital computation or processing), the value of the required illumination intensity in the main illumination period may be computed by the illumination control unit 40A (or by the illumination control unit 40 of Fig. 2).

The computation of the required illumination intensity or of the current required from the LED driver unit 84 may be performed as disclosed hereinabove, using the known value of $I_1$ (see Fig. 10B) and may or may not take into account the duration of the period in which the illuminating unit 63 was turned off. (This duration may be approximately known from the known time required to scan the control pixels 160C and from the approximate time required for the data processing and/or computations). The illumination unit 63 may then be turned on (the turning on is not shown in Fig. 10A for the sake of clarity of illustration) using the computed current value to generate the required illumination intensity value $I_2$ (see Fig. 10B) till the end of the main illumination period 106 at time $T_M$.

It is noted that if the number of control pixels 160C is small the time required for scanning the control pixels 160C may be short in comparison to the total duration of the total illumination period 108. For example, if the scan time for scanning a single control pixel is approximately 6 microseconds, the scanning of **16** control pixels may require about 96 microseconds. Since the time required for computing the required light intensity may also be small (a few microseconds or tens of microseconds may be required), the period of time during which the illumination unit 63 is turned off at the end of the illumination sampling period 104 may comprise a small fraction of the main illumination period 108 which may typically be 20-30 milliseconds.

It may also be possible to compute a weighted average in which the intensity read for each pixel may be differently weighted according to the position of the particular control pixel within the entire pixel array 160. Such weighting methods may be used for obtaining center biased intensity weighting, as is known in the art, or any other type of biased measurement known in the art, including but not limited to edge (or periphery) biased weighting, or any other suitable type of weighting known in the art. Such compensating or weighting computations may be performed by an illumination control unit (not shown) included in the imaging device, or by any suitable processing unit (not shown), or controller unit (not shown) included in the imaging device in which the CMOS imager 160 illustrated in Fig. 13 is included.

Thus, if an averaging or weighting computation is used, after the readout of the control pixels and any type of compensation or weighting computation is finished, the illumination control unit (not shown) may compute the value of the weighted (and/or compensated) quantity of light sensed by the control pixels 160C and use this value for computing the value of $I_2$.

It is noted that the ratio of the number of the control pixels 160C to the regular pixels 160P should be a small number. The ratio of 16/144 which is illustrated is given by example only (for the sake of clarity of illustration). In practical implementations the ratio may be different depending, inter alia, on the total number of pixels in the CMOS array of the imager and on the number of control pixels used. For example in a typical 256 X 256 CMOS pixel array it may be practical to use 16-128 pixels as illumination control pixels for illumination control purposes. The number of control pixels in the 256 X 256 CMOS pixel array may however also be smaller than 16 control pixels or larger than 128 control pixels.

Generally, the number of control pixels and the ratio of control pixels to regular pixels may depend, inter alia, on the total number of pixels available on the imager pixel array, on the pixel scanning speed of the particular imager, on the number of control pixels which may be practically scanned in the time allocated for scanning, and on the duration of the illumination sampling period.

An advantage of the embodiments using automatic illumination control methods in which some of the pixels of the CMOS imager pixel array (such as for example the example illustrated in Fig. 13) is that in contrast to light sensitive sensors which may be disposed externally to the surface of the imager (such as for example, the light sensing unit 67 of Fig. 3), the control pixels 160C actually sense the amount of light reaching the imager's surface since they are also imaging pixels disposed on the surface of the imager. This may be advantageous due to, inter alia, higher accuracy of light sensing, and may also eliminate the need for accurately disposing of the light sensing unit at an optimal place in the optical system, additionally, the control pixels may have signal to noise characteristics and temperature dependence properties similar to the other (non-control) pixels of the imager.

Another advantage of using control pixels is that no external light sensing units are needed which may reduce the cost and simplify the assembly of the imaging device.

It is noted that in a CMOS imager such as the imager 160, the scanning of the control pixels 160C after the illumination sampling period 104 does not reset the pixels. Thus, the control pixels 160C continue to sense the light during the main illumination period 106, and are scanned after the time $T_M$ together with all the other regular pixels 160P of the imager 160. Thus, the acquired image includes the full pixel information since the control pixels 160C and the regular pixels 160P have been exposed to light for the same duration. The image quality or resolution is thus not significantly affected by the use of the control pixels 160C for controlling the illumination.

It is also noted that while the arrangement of the control pixels 160C on the imager 160 is symmetrical with respect to the center of the imager, any other suitable arrangement of the pixels may be used. The number and the distribution of the control pixels on the imager 160 may be changed or adapted in accordance with the type of averaging used.

Furthermore, the control pixels may be grouped into groups which may be processed separately to allow local illumination control in imagers using a plurality of separately controllable light sources.

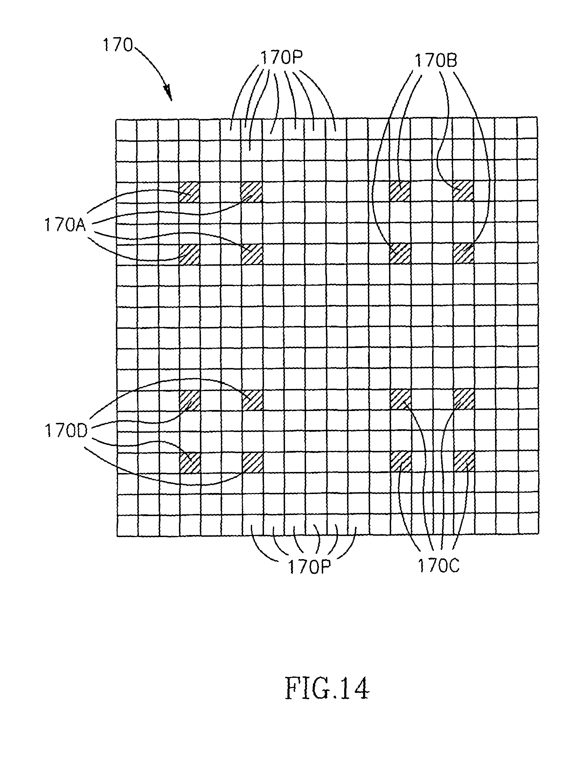

Reference is now made to Fig. 14, which is a schematic top view of the pixels of a CMOS imager illustrating an exemplary distribution of control pixel groups suitable for being used in local illumination control in an imaging device, in accordance with an embodiment of the present invention.

The illustrated imager 170 is a 20X20 pixel array having 400 pixels. The control pixels are schematically represented by the hatched squares 170A, 170B, 170C and 170C and the remaining imager pixels are schematically represented by the non-hatched squares 170P. Four groups of control pixels are illustrated on the imager 170.

The first pixel group includes four control pixels 170A arranged within the top left quadrant of the surface of the imager 170. The second pixel group includes four control pixels 170B arranged within the top right quadrant of the surface of the imager 170. The third pixel group includes four control pixels 170C arranged within the bottom right quadrant of the surface of the imager 170. The fourth pixel group includes four control pixels 170D arranged within the top left bottom quadrant of the surface of the imager 170.

If the imager 170 is disposed in an autonomous imaging device having a plurality of light sources (such as, but not limited to the device 150 of Fig. 12), each of the four groups of control pixels 170A, 170B, 170C and 170D may be scanned and processed as disclosed hereinabove to provide data for locally controlling the illumination level reaching each of the respective four quadrants of the imager 170. The scanned data for each of the pixels within each of the four groups may be processed to compute a desired value of illumination intensity for the respective imager quadrant. The methods for controlling the illumination using separate local control loops may be similar to any of the methods disclosed hereinabove with respect to the device 150 of Fig. 12, except that in the device 150 the light sensing units are units external to the imager and in the device 170, the control pixels used for sensing are imager pixels which are integral parts of the imager 170.

The illumination control methods using control pixels may be implemented using the closed-loop method of terminating the illumination when the integrated sensor signal reaches a threshold level as disclosed hereinabove or may be implemented by using an initial illumination intensity in a sampling illumination period and adapting or modifying the illumination intensity (if necessary) in accordance with a value computed or determined from the control pixel scanning as disclosed hereinabove.

The signals or data of (representing the pixel charge) the pixel groups may be processed using averaging or weighted averaging methods to perform center biased or periphery biased averages or according to any other averaging or processing method known in the art. The results of the processing may be used as disclosed hereinabove to control the light sources (such as for example four light sources disposed within the imaging device in an arrangement similar to the arrangement of the four light sources 163A, 163B, 163C, and 163D of Fig. 12).

It will be appreciated by those skilled in the art that the number of control pixels and the distribution of the control pixels on the surface of the imager may be varied, inter alia, in accordance with the desired type of averaging, the required number of local illumination control groups, the number and position of the light sources available in the imaging device, the computational power available to the processing unit available, the speed of the illumination control unit, and other design considerations.

In accordance with another embodiment of the present invention, the control pixels 160C of Fig. 13 may be specially fabricated pixels which are constructed differently than the regular pixels 160P. In accordance with this embodiment, the control pixels 160C may be fabricated as analog photodiodes with appropriate readout or sampling circuitry (not shown) as is known in the art. This implementation may use a specially fabricated custom CMOS imager in which the analog photodiodes serving as the control pixels 160C may be read simultaneously which may be advantageous since the readout or scanning time may be shorter than the time required to sequentially scan the same number of control pixels implemented in a regular CMOS pixel array having uniform pixel construction.

It is noted that when analog photodiodes or other known types of dedicated sensors are integrated into the CMOS pixel array of the imaging device, the acquired image will have "missing" image pixels, since the area in which the analog photodiode is disposed is not scanned together with the regular CMOS array pixels. The image data will therefore have "missing pixels". If, however, a small number of analog photodiodes or other dedicated control pixels is included in the CMOS pixel array, the missing pixels may not cause a significant degradation of image quality. Additionally, such dedicated analog photodiodes or other control pixels may be distributed within the pixel array and may be sufficiently spaced apart from each other, so that image quality may be only slightly affected by the missing image pixels.

It is noted that while the illumination control methods are disclosed for use in an autonomous imaging device such as the device 10A of Fig. 1, these illumination control methods may also be used with or without adaptations in other in-vivo imaging devices having an imager and an illumination unit, such as in endoscopes or catheter-like devices having imaging sensor arrays, or in devices for performing in-vivo imaging which are insertable through a working channel of an endoscope, or the like.

Additionally, the illumination control methods disclosed herein may be used in still cameras and in video cameras which include a suitable imager, such as a CMOS imager, and which include or are operatively connected to an illumination source.

Additionally, the use of control pixels implemented in a CMOS pixel array imager, using selected regular pixels as control pixels or using specially fabricated control pixels such as the analog photodiodes or the like, may be applied for controlling the illumination of a flash unit or another illumination unit which may be integrated within the camera or may be external to the camera and operatively connected thereto.

The advantages of using control pixels which are part of the CMOS imager of the camera may include, inter alia, simplicity of construction and operation, the ability to implement and use a plurality of controllably interchangeable averaging methods including weighted averaging methods and biasing methods, as disclosed in detail hereinabove, increased accuracy of illumination control.

Additionally, in specialty cameras operating under conditions in which the light source included in the camera or operatively connected thereto is the only source of available illumination (such as, for example, in camera's operated at the bottom of the ocean, or in cameras which are designed to perform surveillance or monitoring in difficult to access areas which are normally dark), the use of illumination control methods disclosed hereinabove may allow the use of shutterless cameras, which may advantageously increase the reliability of such devices, reduce their cost, and simplify their construction and operation.

It is noted that, while in the embodiments of the invention disclosed hereinabove the number and the arrangement of the control pixels are fixed, in accordance with another different embodiment of the present invention, the number and/or the geometrical configuration (arrangement) of the control pixels may be dynamically changed or controlled. For example, briefly turning to Fig. 2, the light sensing unit(s) 42 may represent one or more control pixels of a CMOS pixel array, and the illumination control unit 40, and/or the controller/processor unit 36 may be configured for changing the number of the control pixels used in an imaging acquisition cycle and/or for changing the arrangement of the control pixels on the pixel array of the imaging unit 32.

Such changing of control pixel number and/or arrangement may be performed, in a non-limiting example, by changing number and/or arrangement of the pixels selected to be scanned as control pixels during the illumination sampling period 104 (Fig. 10A). Such changing may allow the use of different averaging arrangements and methods and may allow changing of different biasing methods for different imaging cycles.

Additionally, using dynamically controllable control pixel configuration, it may be possible to implement two or more illumination sampling periods within a single imaging cycle and to use a different pixel number or configuration for each of these two or more illumination sampling periods.

It may also be possible to remotely control the number and/or configuration of the control pixels, by instructions which are wirelessly transmitted to the telemetry unit 34 (Fig. 2), in which case the telemetry unit may be configured as a transceiver unit capable of transmitting data and of receiving control data transmitted to it by an external transmitter unit (not shown in Fig. 2).

It is noted that, while all the embodiments disclosed hereinabove were based on modifying the light output from the illumination unit (such as, for example the illumination unit 63 of Fig. 3) based on measurement and processing of the amount of light reaching the light sensing elements (such as, for example the light sensing unit 67 of Fig. 3, or the light sensing units 42 of Fig. 2, or the control pixels 160C of Fig. 13), another approach may be used. It may be possible to change the gain of the pixel amplifiers (not shown) of the imager based on the results of the measurement of the amount of light reaching the light sensing unit or units (such as, for example, the light sensing unit 67 or the control pixels 160C, or the like). In such an embodiment, the illumination unit of the imaging device(s) (such as, for example, the illumination unit 63 of Fig. 3, or the illumination unit 38 of Fig. 2) may be operated for a fixed time period at a fixed illumination intensity, the light reaching the light sensing unit(s) or the control pixels of the imaging device is measured. The gain or sensitivity of the imager pixel amplifiers may then be changed to achieve proper imaging. For example, if not enough light is reaching the light sensing unit(s) during the illumination sampling period, the pixel amplifier gain may be increased to prevent underexposure. If too much light is reaching the light sensing unit(s) during the illumination sampling period, the pixel amplifier gain may be decreased to prevent overexposure. If the amount of light reaching the light sensing unit(s) during the illumination sampling period is sufficient to ensure proper exposure, the pixel amplifier gain is not changed.

It is noted that such automatic gain control may result, under certain conditions in changes in the signal to noise ratio (S/N) of the imager in some cases. For example, increasing the pixel amplifier gain in CMOS pixel array imagers may result in lower S/N ratio.

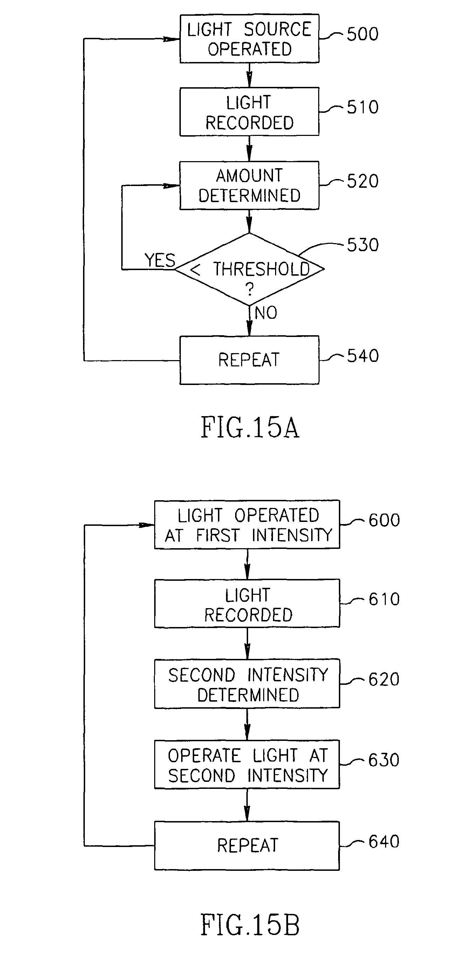

Fig. 15A depicts a series of steps of a method according to an embodiment of the present invention. In alternate embodiments, other steps, and other series of steps, may be used.

In step 500, a device, such as an in-vivo imaging device, turns on a light source.

In step 510, the device records the amount of light received to the device or to a sensor. This may be, for example, to a sensor on the device, or possibly to an external sensor.

In step 520, the device determines the amount of light recorded.

In step 530, if the amount of light recorded is less than a threshold, the device method repeats step 520; if not, the method continues to step 540.

In step 540, the method repeats at step 500, as, typically, the device operates across a series of imaging periods. However, the method need not repeat.

Fig. 15B depicts a series of steps of a method according to an alternate embodiment of the present invention. In further embodiments, other steps, and other series of steps, may be used.

In step 600, a device, such as an in-vivo imaging device, turns on a light source at a first intensity. The light is typically operated for a first fixed period, e.g., a sampling period.

In step 610, the device records the amount of light received by the device or to a sensor while the light source is operated at the first intensity. The recording may be, for example, of the light received to a sensor on the device, or possibly to an external sensor.

In step 620, the device determines the intensity for the operation of the light during a second period. This determination may be, for example, designed to ensure the probability that during both the first and second periods, the total amount of light received is within a certain range or near a certain target. Other methods of determining the intensity may be used

In step 630, the light is operated at the second intensity. The light is typically operated for a second fixed period.

In step 640, the method repeats at step 600, as, typically, the device operates across a series of imaging periods. However, the method need not repeat.

**Claims**

1. A swallowable in vivo video imaging capsule (30) comprising:

   a light source (38);
   an imager (32); and
   a controller (40),
   **characterized in that** the controller (40) is configured to, during several imaging periods ($\Delta T1$) that each comprise an illumination period (90) and a dark period (92), where imaging periods occur at a rate of 2-8 per second:
   operate the light source (38), record the amount of light reflected to the imaging capsule and, based on when a certain amount of light is recorded, switch off the light source.

2. The imaging capsule of claim 1 wherein the controller is configured to record the amount of light based on a signal from the imager.

3. The imaging capsule of claim 1 wherein the imager is a CMOS imager.

4. The imaging capsule of claim 1 comprising a light sensor (42), and wherein the controller is configured to record the amount of light reflected based on a signal from the light sensor (42).

5. The imaging capsule of claim 4 wherein the light sensor is a photodiode.

6. The imaging capsule of claim 1 wherein the amount of light reflected is the cumulative amount of light reflected.

7. The imaging capsule of claim 1 comprising a transmitter (26).

8. The imaging capsule of claim 1 comprising a battery (44).

9. The imaging capsule of claim 1 wherein the light source includes an LED.

10. The imaging capsule of claim 1 wherein the light source includes a plurality of discrete light sources (38A-38N).

11. The imaging capsule of claim 1 wherein the light source includes a plurality of discrete light sources (130A-130N), the imager comprising a plurality of discrete light sensors (122A-122N), each of a plurality of sets of light sensors (122A-122N) being paired with a set among a plurality of sets of discrete light sources (130A-130N), wherein the controller is configured to, for each pair of light sensor set and light source set, operate the set of light sources, record the amount of light reflected to the light sensor set, and, when a certain amount of light is recorded, switch off the light source set.

12. The imaging capsule of claim 1 wherein the controller is configured to operate over a series of imaging periods, and, during each imaging period, to acquire an image from the imager.

13. The imaging capsule of claim 1 wherein the controller is configured to, during an imaging period, switch off the light source when a time limit is reached.

14. The imaging capsule of claim 1 wherein the imaging period is varied according to a sensed velocity of the imaging capsule.

15. A method for operating a swallowable in vivo video imaging capsule (30) including a light source (38), the method comprising:

during several imaging periods (ΔT1) that each comprise an illumination period (90) and a dark period (92), where imaging periods occur at a rate of 2-8 per second:
operating the light source (38);
recording the amount of light reflected to the imaging capsule; and
based on when a certain amount of light is recorded, switching off the light source (38).

16. The method of claim 15 wherein the device includes an imager.

17. The method of claim 16 comprising recording the amount of light based on a signal from the imager.

18. The method of claim 16 wherein the imager is a CMOS imager.

19. The method of claim 15 wherein the device comprises a light sensor, the method comprising recording the amount of light reflected based on a signal from the light sensor.

20. The method of claim 19 wherein the light sensor is a photodiode.

21. The method of claim 15 wherein the amount of light reflected is the cumulative amount of light reflected.

22. The method of claim 15 wherein the light source includes a plurality of discrete light sources.

23. The method of claim 15 wherein the light source includes a plurality of discrete light sources, the imaging device comprising a plurality of discrete light sensors, each of a plurality of sets of light sensors being paired with a set among a plurality of sets of discrete light sources, the method comprising:

for each pair of light sensor set and light source set:
operating the set of light sources;
recording the amount of light reflected to the light sensor set; and
when a certain amount of light is recorded, switching off the light source set.

24. The method of claim 15 wherein the device operates over a series of imaging periods, the method comprising, during each imaging period, acquiring an image from the imager.

25. The method of claim 15 comprising, during an imaging period, switching off the light source when a time limit is reached.

26. The method of claim 15 wherein the imaging period is varied according to a sensed velocity of the imaging capsule.

**Patentansprüche**

1. Verschluckbare In-Vivo-Videobildkapsel (30), mit:

einer Lichtquelle (38);
einem Bildgerät (32); und
einem Steuergerät (40),
**dadurch gekennzeichnet, dass** das Steuergerät (40) konfiguriert ist, während mehrerer Bildzeiträume (ΔT1), die alle einen Beleuchtungszeitraum (90) und einen Dunkelzeitraum (92) umfassen, wobei die Bildzeiträume bei einer Rate von 2-8 je Sekunde auftreten:
die Lichtquelle (38) zu betreiben, die Menge von Licht aufzunehmen, das zu der Bildkapsel reflektiert wird, und basierend darauf, wenn eine bestimmte Menge von Licht aufgenommen wird, die Lichtquelle auszuschalten.

2. Bildkapsel nach Anspruch 1, wobei das Steuergerät konfiguriert ist, die Menge von Licht basierend auf einem Signal von dem Bildgerät aufzunehmen.

3. Bildkapsel nach Anspruch 1, wobei das Bildgerät ein CMOS-Bildgerät ist.

4. Bildkapsel nach Anspruch 1, mit einem Lichtsensor (42) und wobei das Steuergerät konfiguriert ist, die Menge von reflektiertem Licht basierend auf einem Signal von dem Lichtsensor (42) aufzunehmen.

5. Bildkapsel nach Anspruch 4, wobei der Lichtsensor eine Photodiode ist.

6. Bildkapsel nach Anspruch 1, wobei die Menge eines reflektierten Lichtes die kumulative Menge von reflektiertem Licht ist.

7. Bildkapsel nach Anspruch 1, mit einem Sender (26).

8. Bildkapsel nach Anspruch 1, mit einer Batterie (44).

9. Bildkapsel nach Anspruch 1, wobei die Lichtquelle eine LED einschließt.

10. Bildkapsel nach Anspruch 1, wobei die Lichtquelle eine Vielzahl von getrennten Lichtquellen (38A-38N) einschließt.

11. Bildkapsel nach Anspruch 1, wobei die Lichtquelle eine Vielzahl von getrennten Lichtquellen (130A-130N) einschließt, das Bildgerät eine Vielzahl von getrennten Lichtsensoren (122A-122N) einschließt, wobei jeder einer Vielzahl von Sätzen von Lichtsensoren (122A-122N) mit einem Satz aus einer Vielzahl von Sätzen von getrennten Lichtquellen (130A-130N) gepaart ist, wobei das Steuergerät konfiguriert ist, für jedes Paar eines Lichtsensorsatzes und eines Lichtquellensatzes den Satz von Lichtquellen zu betreiben, die Menge von Licht aufzunehmen, das zu dem Lichtsensorsatz reflektiert wird, und basierend darauf, wenn eine bestimmte Menge von Licht aufgenommen wird, den Lichtquellensatz auszuschalten.

12. Bildkapsel nach Anspruch 1, wobei das Steuergerät konfiguriert ist, über eine Serie von Bildzeiträumen zu arbeiten und während jedem Bildzeitraum ein Bild von dem Bildgerät zu erfassen.

13. Bildkapsel nach Anspruch 1, wobei das Steuergerät konfiguriert ist, während einem Bildzeitraum die Lichtquelle abzuschalten, wenn eine Zeitgrenze erreicht ist.

14. Bildkapsel nach Anspruch 1, wobei der Bildzeitraum gemäß einer abgetasteten Geschwindigkeit der Bildkapsel variiert wird.

15. Verfahren zum Betreiben einer verschluckbaren In-Vivo-Videobildkapsel (30) einschließlich einer Lichtquelle (38), wobei das Verfahren umfasst:

während mehrerer Bildzeiträume ($\Delta T1$), die alle einen Beleuchtungszeitraum (90) und einen Dunkelzeitraum (92) umfassen, wobei die Bildzeiträume bei einer Rate von 2-8 je Sekunde auftreten:
Betreiben der Lichtquelle (38);
Aufnehmen der Menge von Licht, das zu der Bildkapsel reflektiert wird; und
basierend darauf, wenn eine bestimmte Menge von Licht aufgenommen wird, Ausschalten der Lichtquelle (38).

16. Verfahren nach Anspruch 15, wobei das Gerät ein Bildgerät einschließt.

17. Verfahren nach Anspruch 16, mit einem Aufnehmen der Menge von Licht basierend auf einem Signal von dem Bildgerät.

18. Verfahren nach Anspruch 16, wobei das Bildgerät ein CMOS-Bildgerät ist.

19. Verfahren nach Anspruch 15, wobei das Gerät einen Lichtsensor umfasst, wobei das Verfahren ein Aufnehmen der Menge von reflektiertem Licht basierend auf einem Signal von dem Lichtsensor umfasst.

20. Verfahren nach Anspruch 19, wobei der Lichtsensor eine Photodiode ist.

**21.** Verfahren nach Anspruch 15, wobei die Menge eines reflektierten Lichtes die kumulative Menge von reflektiertem Licht ist.

**22.** Verfahren nach Anspruch 15, wobei die Lichtquelle eine Vielzahl von getrennten Lichtquellen (38A-38N) einschließt.

**23.** Verfahren nach Anspruch 15, wobei die Lichtquelle eine Vielzahl von getrennten Lichtquellen einschließt, das Bildgerät eine Vielzahl von getrennten Lichtsensoren einschließt, wobei jeder einer Vielzahl von Sätzen von Lichtsensoren mit einem Satz aus einer Vielzahl von Sätzen von getrennten Lichtquellen gepaart ist, wobei das Verfahren umfasst:

> für jedes Paar eines Lichtsensorsatzes und eines Lichtquellensatzes:
> Betreiben des Satzes von Lichtquellen;
> Aufnehmen der Menge von Licht, das zu der Bildkapsel reflektiert wird; und
> wenn eine bestimmte Menge von Licht aufgenommen wird, Ausschalten des Lichtquellensatzes.

**24.** Verfahren nach Anspruch 15, wobei das Gerät über eine Serie von Bildzeiträumen arbeitet, wobei das Verfahren während jedem Bildzeitraum ein Erfassen eines Bildes von dem Bildgerät umfasst.

**25.** Verfahren nach Anspruch 15, mit einem Ausschalten der Lichtquelle während einem Bildzeitraum, wenn eine Zeitgrenze erreicht ist.

**26.** Verfahren nach Anspruch 15, wobei der Bildzeitraum gemäß einer abgetasteten Geschwindigkeit der Bildkapsel variiert wird.

**Revendications**

**1.** Capsule (30) d'imagerie vidéo in vivo pouvant être avalée, comprenant :

> une source lumineuse (38);
> un imageur (32); et
> une unité de commande (40),
> **caractérisée en ce que** l'unité de commande (40) est configurée de manière à, durant plusieurs périodes d'imagerie ($\Delta T1$) qui comprennent chacune une période d'illumination (90) et une période sombre (92), où les périodes d'imagerie se produisent à une cadence de 2-8 par seconde :
> mettre en fonctionnement la source lumineuse (38), enregistrer la quantité de lumière réfléchie au niveau de la capsule d'imagerie et, lorsqu'une certaine quantité de lumière est enregistrée, arrêter le fonctionnement de la source lumineuse.

**2.** Capsule d'imagerie de la revendication 1 dans laquelle l'unité de commande est configurée de manière à enregistrer la quantité de lumière sur la base d'un signal provenant de l'imageur.

**3.** Capsule d'imagerie de la revendication 1 dans laquelle l'imageur est un imageur CMOS.

**4.** Capsule d'imagerie de la revendication 1 comprenant un capteur (42) de lumière, et où l'unité de commande est configurée de manière à enregistrer la quantité de lumière réfléchie sur la base d'un signal provenant du capteur (42) de lumière.

**5.** Capsule d'imagerie de la revendication 4 dans laquelle le capteur de lumière est une photodiode.

**6.** Capsule d'imagerie de la revendication 1 dans laquelle la quantité de lumière réfléchie est la quantité cumulée de lumière réfléchie.

**7.** Capsule d'imagerie de la revendication 1 comprenant un émetteur (26).

**8.** Capsule d'imagerie de la revendication 1 comprenant une batterie (44).

**9.** Capsule d'imagerie de la revendication 1 dans laquelle la source lumineuse comporte une diode électroluminescente.

**10.** Capsule d'imagerie de la revendication 1 dans laquelle la source lumineuse comporte une pluralité de sources lumineuses discrètes (38A-38N).

**11.** Capsule d'imagerie de la revendication 1 dans laquelle la source lumineuse comporte une pluralité de sources lumineuses discrètes (130A-130N), l'imageur comprenant une pluralité de capteurs (122A-122N) de lumière discrète, chacun d'une pluralité d'ensembles de capteurs (122A-122N) de lumière étant apparié avec un ensemble parmi une pluralité d'ensembles de sources lumineuses discrètes (130A-130N), où l'unité de commande est configurée de manière à, pour chaque paire d'ensemble de capteurs de lumière et d'ensemble de sources lumineuses, mettre en fonctionnement l'ensemble de sources lumineuses, enregistrer la quantité de lumière réfléchie au niveau de l'ensemble de capteurs de lumière, et, lorsqu'une certaine quantité de lumière est enregistrée, arrêter le fonctionnement de l'ensemble des sources lumineuses.

**12.** Capsule d'imagerie de la revendication 1 dans laquelle l'unité de commande est configurée de manière à fonctionner sur une série de périodes d'imagerie, et, durant chaque période d'imagerie, à acquérir une image provenant de l'imageur.

**13.** Capsule d'imagerie de la revendication 1 dans laquelle l'unité de commande est configurée de manière à, durant une période d'imagerie, mettre la source lumineuse à l'arrêt lorsqu'une limite de temps est atteinte.

**14.** Capsule d'imagerie de la revendication 1 dans laquelle la période d'imagerie varie selon la vitesse détectée de la capsule d'imagerie.

**15.** Procédé pour mettre en service une capsule (30) d'imagerie vidéo in vivo pouvant être avalée comportant une source lumineuse (38),

le procédé comprenant :
durant plusieurs périodes d'imagerie ($\Delta$T1) qui comprennent chacune une période d'illumination (90) et une période sombre (92), où des périodes d'imagerie se produisent à une cadence de 2-8 par seconde, le fait de :
faire fonctionner la source lumineuse (38);
enregistrer la quantité de lumière réfléchie au niveau de la capsule d'imagerie; et
lorsqu'une certaine quantité de lumière est enregistrée, arrêter le fonctionnement de la source lumineuse (38).

**16.** Procédé de la revendication 15 dans lequel le dispositif comporte un imageur.

**17.** Procédé de la revendication 16 comprenant l'enregistrement de la quantité de lumière sur la base d'un signal provenant de l'imageur.

**18.** Procédé de la revendication 16 dans lequel l'imageur est un imageur CMOS.

**19.** Procédé de la revendication 15 dans lequel le dispositif comprend un capteur de lumière, le procédé comprenant l'enregistrement de la quantité de lumière réfléchie sur la base d'un signal provenant du capteur de lumière.

**20.** Procédé de la revendication 19 dans lequel le capteur de lumière est une photodiode.

**21.** Procédé de la revendication 15 dans lequel la quantité de lumière réfléchie est la quantité cumulée de lumière réfléchie.

**22.** Procédé de la revendication 15 dans lequel la source lumineuse comporte une pluralité de sources lumineuses discrètes.

**23.** Procédé de la revendication 15 dans lequel la source lumineuse comporte une pluralité de sources lumineuses discrètes, le dispositif d'imagerie comprenant une pluralité de capteurs de lumière discrète, chacun d'une pluralité d'ensembles de capteurs de lumière étant apparié à un ensemble parmi une pluralité d'ensembles de sources lumineuses discrètes, le procédé comprenant :

pour chaque paire d'ensemble de capteurs de lumière et d'ensemble de sources lumineuses, le fait de :
faire fonctionner l'ensemble de sources lumineuses;
enregistrer la quantité de lumière réfléchie au niveau de l'ensemble de capteurs de lumière; et

lorsqu'une certaine quantité de lumière est enregistrée, arrêter le fonctionnement de l'ensemble de sources lumineuses.

24. Procédé de la revendication 15 dans lequel le dispositif fonctionne sur une série de périodes d'imagerie, le procédé comprenant, durant chaque période d'imagerie, l'acquisition d'une image provenant de l'imageur.

25. Procédé de la revendication 15 comprenant, durant la période d'imagerie, le fait de mettre la source lumineuse à l'arrêt lorsqu'une limite de temps est atteinte.

26. Procédé de la revendication 15 dans lequel la période d'imagerie varie selon la vitesse détectée de la capsule d'imagerie.

FIG.1

FIG.2

FIG.3

FIG.4

EP 1 411 818 B1

FIG.5

FIG.6

FIG.7

FIG.8

FIG.9

FIG.10A

FIG.10B

FIG.10C

FIG.11

FIG.12

FIG.13

FIG.14

LIGHT SOURCE OPERATED — 500

↓

LIGHT RECORDED — 510

↓

AMOUNT DETERMINED — 520

↓

< THRESHOLD ? — 530

YES

NO

REPEAT — 540

FIG.15A

LIGHT OPERATED AT FIRST INTENSITY — 600

↓

LIGHT RECORDED — 610

↓

SECOND INTENSITY DETERMINED — 620

↓

OPERATE LIGHT AT SECOND INTENSITY — 630

↓

REPEAT — 640

FIG.15B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6240312 B
- US 5604531 A, Iddan
- US 2001035902 A, Glukhovsky
- US 800470 A, Glukhovsky
- US 6709387 B